# EUROPEAN PATENT APPLICATION

(11) **EP 1 158 000 A1**
(43) Date of publication of application: **28.11.2001**
(21) Application number: 00111121.0
(22) Date of filing: 23.05.2000
(51) Int. Cl.: C07K 14/20, A61K 31/198, A61P 1/02, G06F 17/50

(54) **Crystal structure data of cystalysin and use thereof for the design and preparation of inhibitor molecules**

(71) Applicant: Max-Planck-Gesellschaft zur Förderung der Wissenschaften e.V., 80539 München (DE)
(72) Inventor: Claussen, Tim, Dr., 80337 München (DE); Krupka, Heike, 85521 Ottobrunn (DE); Huber, Robert, Prof., 82110 Germering (DE)
(74) Representative: Böhm, Brigitte, Dipl.-Chem. Dr.

(57) **Abstract**

A crystalline preparation of cystalysin is used to create crystal structure data. Such crystal structure data is used for the design and/or identification and/or preparation of inhibitors, which specifically inhibit cystalysin activity. Such inhibitors and pharmaceutical compositions containing such inhibitors are further subjects of the present invention.

## Description

The present invention concerns the crystal structure of cystalysin from *Treponema denticola,* the use of said crystal structure in order to obtain crystal structure data enabling rational drug design and inhibitors of cystalysin, pharmaceutical compositions containing such inhibitors as well as the use of said pharmaceutical compositions for the treatment and prevention of periodontal diseases.

Cystalysin is a 45 kDa pyridoxal 5'-phosphate (PLP)-protein isolated from *Treponema denticola* (Chu and Holt, 1994). This organism belongs to the group of oral spirochetes, that are located in the human gingival crevice. Especially high concentrations of treponemes have been found at diseased sites in the subgingiva of patients with severe periodontitis. Evidence is increasing that oral treponemes play an important role in disease progression leading to gingival inflammation and bleeding, deattachment of connective tissue, formation of lesions and abscesses, and finally to tissue and bone destruction (Chu & Holt, 1994; Chu et al., 1997).

Since *T. denticola* appears to be restricted to the ecological niche of periodontal pockets, it had to develop special techniques to cope with an environment that requires complex nutritional needs. In vitro studies revealed the production of a large number of virulence factors by *T. denticola,* including several proteolytic and cytotoxic enzymes. The equipment with such potential tools for cell-adhesion and degrading of host proteins/peptides seems to be a prerequisite for bacterial growth and the progression of periodontal lesions (Chu and Holt, 1994).

*T. denticola* mainly produces one putative hemolysin, that is able to agglutinate and lyse erythrocytes upon incubation. By studying the hemolytic and hemoxidizing properties of *T. denticola* strain TD-4, Holt and coworkers could pinpoint both activities to a cytoplasmic 46 kDa protein (Chu et al., 1994). Both the native and the recombinant cystalysin were able to interact with human and sheep red blood cells (Chu & Holt, 1994) leading to impressive spikes and protrusions in the erythrocyte membranes and finally to the formation of countless irregular holes (Chu et al., 1994). Additionally, cystalysin causes the oxidation and sulfuration of hemoglobin to methemoglobin and sulfhemoglobin (Kurzban et al., 1999), respectively. The cytoplasm leaking from the lysed red blood cells contains a large number of nutrition factors suitable for *T. denticola,* especially various amino acids and the detached heme from hemoglobin. With the iron from extracellular heme, a rather scarce substance of the periodontal pocket becomes available for T. denticola. The central role of cystalysin in iron acquisition is further supported by the observation that cystalysin synthesis is significantly increased under conditions of iron deprivation (Chu et al., 1994).

Studies of the amino acid sequence of cystalysin (Chu et al., 1995) revealed no relation to previously reported hemolytic proteins but showed significant homologies with the family of PLP-dependent aminotransferases. This suggests that hemolysis as performed by cystalysin is proceeding by a novel, unique mechanism.

PLP could be identified as cofactor of cystalysin (Chu et al., 1995), forming a Schiff base with the ε-amino group of the active site lysin (Lys238). Due to the nature of the PLP cofactor, the enzymatic activity was tested for a large number of amino acids (Chu et al., 1997; Kurzan et al., 1999). Only with thiol-derived functional groups including cystathione, reduced glutathione, cystine and cysteine were metabolized by cystalysin. Among these substrates, cysteine was cleaved with highest efficiency (K_{M} = 3.6 mM, K_{cat} = 12 s⁻¹) (Chu et al., 1997). The C^{β}-S^{*Y*} cleavage catalyzed by cystalysin with cysteine, cystathionine and cystine are α, β elimination reactions, yielding ammonia, pyruvate, H₂S, homocysteine and cysteine desulfhydrase (Chu et al., 1997) catalyzing reactions similar to cystathionine β-lyase (CBL) (Dwivedi et al., 1982), L-cysteine/cystine C-S-lyase from *Synechocystis* (Leibrecht et al., 1997) and MalY (Zdych et al., 1995).

Interestingly, comparably high levels (> 2 mM) of H₂S have been reported in periodontal disease pockets. In these concentrations H₂S is toxic for most cells, since it is able to cleave disulfide bonds in proteins, and to inactivate metalloproteins. It is also known to be a potent inhibitor of oxidative phosphorylation (Beauchamp et al., 1984). T. denticola belongs to a limited number of periodontal microbes that can produce and tolerate millimolar levels of H₂S (Persson et al., 1990). This ability should give selective advantages to T. denticola in its natural environment. For this reason the production of H₂S should be the major function of cystalysin, which thus can be regarded as an important virulence factor (Chu et al., 1997; Kurzban et al., 1999).

In view of the role which cystalysin plays in the generation and the continuance of periodontitis and in view of the fact that is seems very desirable to provide possibilities of controlling periodontitis by destroying the agents causing the disease, the object of the present invention was to find out more about the conformation of the protein cystalysin as well as the sites of interaction with its substrates and, hence, to provide the possibility to identify and/or produce potent inhibitors for this protein.

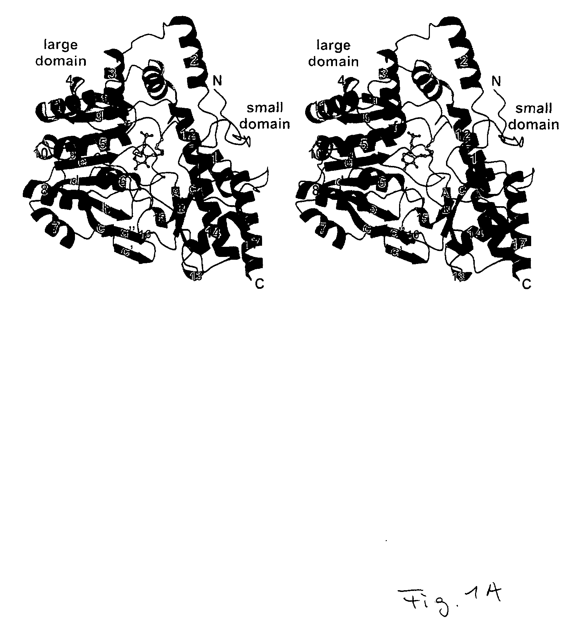

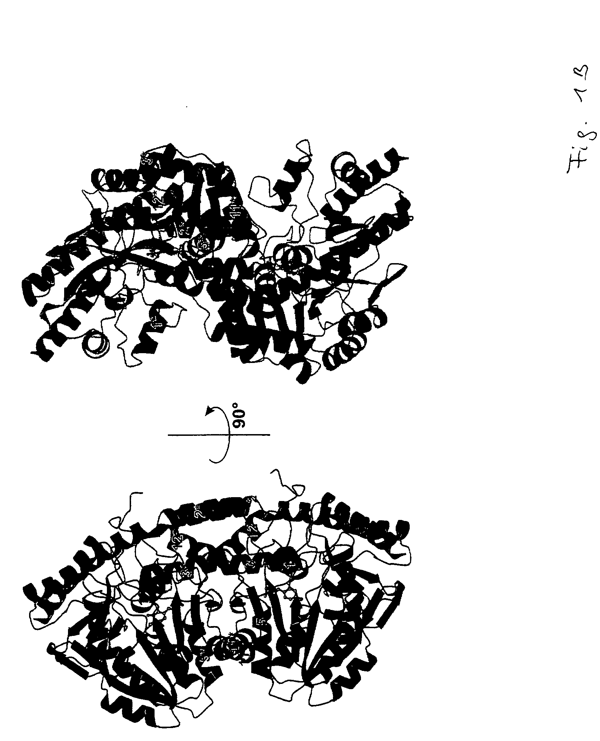

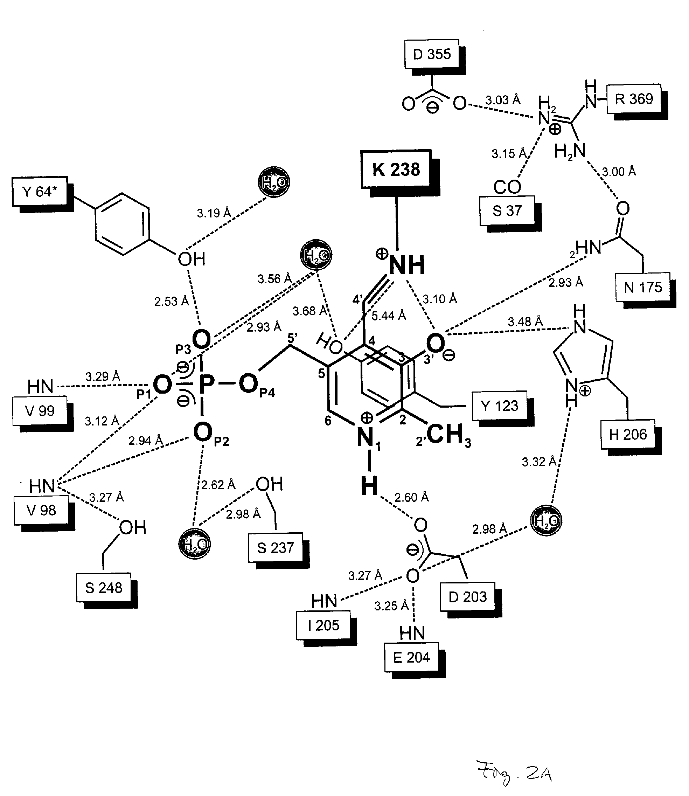

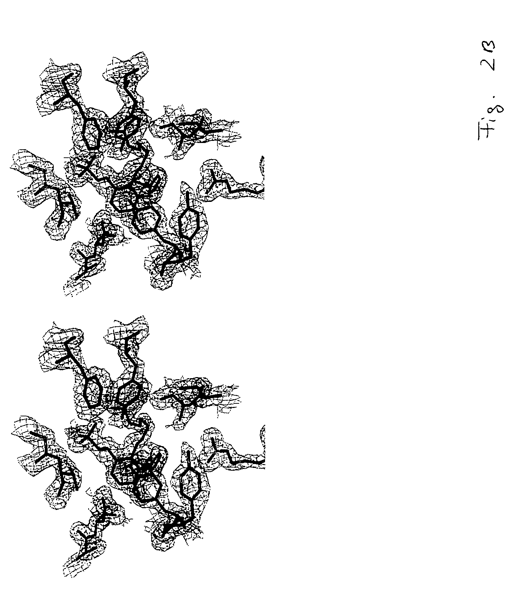

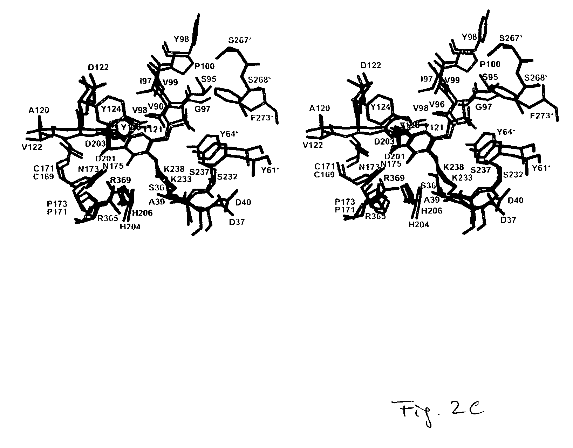

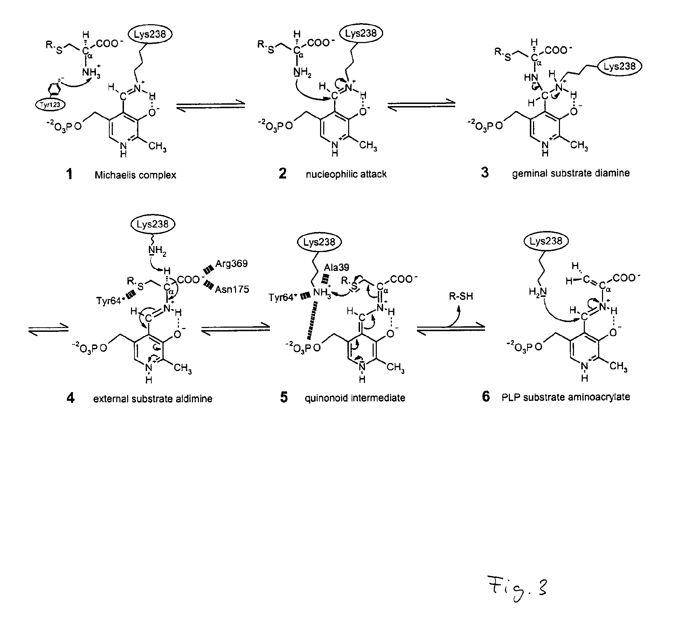

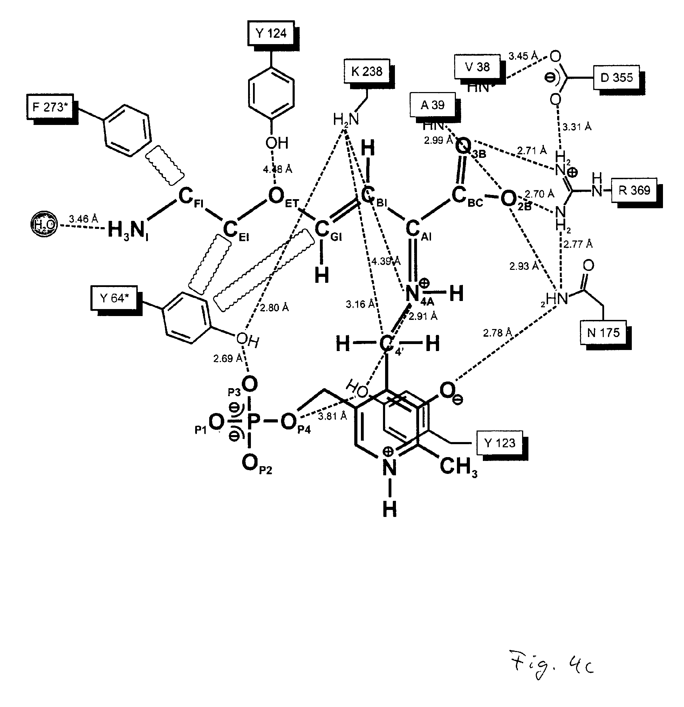

This object is solved by providing a crystalline preparation of cystalysin with the crystal and molecular structure as shown in Fig. 1. The object is further solved by providing a crystalline preparation of a complex of cystalysin and L-aminoethoxyvinylglycine (AVG), a molecule that specifically interacts with cystalysin, the complex being shown in Fig. 4, and therefrom deriving the active site structure as shown in Fig. 2.

The crystal and molecular structures are further described in the following detailed disclosure of the invention.

The structure of cystalysin was solved by molecular replacement to 1.9 Å resolution. As a search model the recently solved structure of the PLP-dependent MalY protein from E. coli was used (Clausen et al., 2000). This enzyme is with 27 % sequence identity the most homologous protein with known crystal structure. The electron density contoured at 1.0 σ is continuous throughout the structure including the PLP cofactor. Only side chains of the surfacial lysins Lys154, Lys161, Lys 163 and Lys184 could not be incorporated in the density. The final model of cystalysin consists of all 394 residues and 328 water molecules per monomer and was refined to a crystallographical R-factor of 20.8 % and a free R-factor of 24.7 %. The model of the cystalysin-AVG complex has a final R-factor of 19.3 % (R_{free} 26.0%). The mean error of atom positions as determined from a Luzzatti plot (Luzatti, 1952) were 0.35 Å. In the Ramachandran plot (Ramachandran and Saisekharan, 1968), 88.0 % of the residues were found to be the most favorable, 11.7 % in the favorable, 0.3 % in the generously allowed and none in the disallowed region as indicated by the program PROCHECK (Laskowski et al., 1993). During the refinement the model bias was eliminated by 8-fold averaging and simulated annealing. The general correctness of the solution was further attested by the 'omit' density which appeared for the PLP cofactor.

Analysis of the secondary structure of cystalysin with the programs STRIDE (Frischman and Argos, 1995) and DSSP (Kabsch and Sander, 1983) revealed an open α/β type architecture with 46.2 % helical structure, 14.2 % β-sheet, 25.4 % β-bend and 14.2 % unclassified coil structure. Within the helical content there are five 3₁₀ helices (3, 9, 13, 15 and 16) three or four residues long. The secondary structural elements are depicted in Figure 1A along with the used nomenclature.

Similarly to most PLP-enzyme structures each cystalysin monomer folds into two domains (Figure 1A) and has an approximate size of 67 Å x 62 Å x 50 Å. The large domain is constituted of residues 48 to 288 and carries the PLP cofactor covalently bound to Lys238. The small domain is assembled by the two terminal regions of the polypeptide chain, by residues 1 to 47 from the N-terminal and residues 288 to 394 from the C-terminal part. The central element of the large domain is a mainly parallel 7-stranded β-sheet that is found in all α-family aminotransferases (Jansonius, 1998). Except for the antiparallel β-strand g, all strands run parallel and form a β-sheet with a left-handed twist enveloping helix 5. All cross-overs are right handed. Additionally, strands c' and c" following β-sheet c, form an antiparallel 2-stranded β-sheet at the interface of both domains. Both sheets are wedged between five helices (4, 7, 8, 9 and 10) at the solvent accessible side, one (6) at the intersubunit and four helices (2, 3, 5 and 11) at the interdomain interfaces. Helices 2 and 11 lead from or into the small domain formed by N-terminal and C-terminal segments, respectively. The long α-helix 12 (39 residues) with a kink at Cys288 is the most characteristic secondary structural element of the model. The small domain is formed by a 3-stranded, antiparallel β-sheet that is protected from solvent by four α-helices (1, 12, 14 and 17) and one 3₁₀ helix (13). α-Helix 1 (residues 17 to 25) is contributed by the N-terminal segment which was found to be well ordered and mainly involved in dimer contacts. Two additional 3₁₀ helices (15 and 16) are forming part of the interdomain interface due to interactions with the loop connecting β-strand b with helix 6 from the large domain. Both domains form a deep cleft in the center of the cystalysin monomer, at the bottom of which the PLP cofactor is bound to the C-terminal end of the 7-stranded β-sheet.

As deduced from gelfiltration experiments, cystalysin occurs as a homodimer of 95 kDa in solution. This quarternary assembly was also observed in the crystalline state. The two monomers are tightly associated through a non-crystallographic 2-fold axis, resulting in an overall arch-shaped appearance (Figure 1B). Figure 1B shows the dimer with one monomer displaying the active site entrance, while the other active site cleft is oriented in opposite direction. In this orientation the αβα-architecture of the dimer is clearly visible, with the two main β-sheet assembled like a winding ladder in the center of the molecule. Rotating the molecule about 90° demonstrates the S-shaped dimer typical for this family of PLP-enzymes (Figure 1B).

Although the dimer displays a rather open structure, the contact surface between the monomers is extensive: approximately 11.2 % (1906 Å) of the total solvent-accessible area of a monomer is buried upon dimerization. Only considering contacts shorter than 3.8 Å, a total of 16 hydrogen bonds and 2 salt bridges participates in subunit interactions. Dimer formation is mediated mainly by two α-helix bundles per monomer (2/3, 11/12 and 2*/3*, 11*/12*). Each helix-bundle is connected by a loop that is reaching into the neighbouring subunit, the loop between helix 2 and 3 even protruding into the adjacent active site. Consequently, residue Tyr64, situated within this loop is directly involved in binding of the PLP cofactor (see below) of the other subunit. Further dimer stabilization is achieved by the N-terminal coil structure and by helices 1 and 5 that interact with the loop between helices 2* and 3* and the C-terminal part of helix 11*, respectively. Additionally, large hydrophobic patches are formed between the helices 2 and 2* which are positioned in almost perfect antiparallel orientation. The large contact surface with numerous interactions between monomers support the notion that the physiologically active state of the cystalysin enzyme is the dimer.

For three-dimensional structural comparisons the DALI algorithm (Holm and Sander, 1993) was employed. The three highest scoring structures of the PDB database are aspartate aminotransferase from *Thermus thermophilus* (Nakai et al., 199) (tAAT, z-score 40.1), tyrosine aminotransferase from *Trypanosoma cruzi* (Blankenfeldt et al., 1999) (z-score 38.4) and 8-amino-7-oxononate synthase from E. coli (Alexeev et al., 1998) (z-score 23.2). tAAT and MalY from E. coli (was not taken into account by the DALI server, since the coordinates had not yet been deposited) were used for more detailed structural comparison.

PLP-dependent enzymes have been classified into three families (α-, β-, and γ-family) with subdivision of the α-family into subgroups I-IV (Metha et al., 1993; Käck et al., 1999). According to Okamoto et al. (Okamoto et al., 1996) subgroup I was further subdivided into subgroups la and lb. 8-amino-7-oxononate synthase and tyrosine aminotransferase belong to the α-family, with the latter being assigned to subgroup I. The aspartate aminotransferases (AATs) belong to subgroup la (e.g. E. coli, yeast, chicken and pig AAT (pAAT) (Rhee et al., 1997) or subgroup lb (e.g. Bacillus sp. (Sung et al., 1991), *T. thermophilus* (Nakai et al., 1999) and *Rhizobium meliloti* AAT (Watson and Rastogi; 1993)), sharing over 40 % sequence identity within each group but only 16 % or less between both subgroups. The C^{α}-alignment of cystalysin with AATs revealed clearly higher similarities with subgroup lb than with subgroup la; with tAAT from *T. thermophilus* 357 C^{α} align (r.m.s deviation 2.3 Å) compared to pAAT (closed form), with 279 aligned C^{α} (r.m.s deviation 3.3 Å).

The striking difference between the two aminotransferase subgroups is the ability of AATs in subgroup la to undergo a large conformational change from the open to a closed form, induced by the binding of substrates or inhibitors (Jansonius, 1998, Rhee et al., 1997). Not surprisingly, typical residues like Gly36, Val37, Gly38, Met326, and the substrate binding Arg292 (Rhee et al., 1997) that enable a transition from the open to the closed conformation of pAAT are absent in cystalysin. In AATs of subgroup lb only the N-terminal region (Lys13-Val30) of the small domain moves to close the active site upon substrate binding (Nakai et al., 1999). Remarkably, the construction and location of the N-terminal segment differs strongly between cystalysin and AATs of this subgroup. In tAAT only 11 residues form a short arm, while the N-terminus of cystalysin contains an α-helix (Leu17-Gln25) among coil structures with a total of 48 residues. Furthermore, the high flexibility of the respective N-terminal segments suggests a general role of this domain for active site closure of subgroup lb AATs and may also be relevant for cystalysin. However, even in the enzyme-inhibitor complex of cystalysin with AVG such a conformational change has not been observed. Overall, the obvious structural similarities with tAAT and MalY (see below) lead to the classification of cystalysin into subgroup lb.

The structure is more similar to MalY (355 C^{α} aligned, r.m.s deviation 1.7 Å) than to tAAT in the entire molecule. Differences are mainly restricted to two regions, which are unique for both enzymes and should play an important role for their respective physiological functions: (1) The structural components that contribute to the individual substrate binding pockets vary in length and architecture generating different morphologies of the catalytic clefts with divergent electrostatic surface topologies. The corresponding active site boundaries can be divided into a proximal part (helix 14 and its C-terminal loop) and a distal part (the N-terminal segment, helix* and its C-terminal loop), relative to their location to the PLP cofactor. While in MalY the active site accessibility via the proximal part is more pronounced, the substrate-binding pocket of cystalysin is more open in the distal half. The different morphology is paralleled by an inverse active site polarity: while the proximal part of the MalY active site is constructed mainly by hydrophobic residues (Phe25, Leu340, Met351, Tyr354), cystalysin shows a highly acidic surface formed by Gln25, Glu343, Asp355, Glu356 and Tyr358. A similar opposing behaviour is observed in the distal half of the active site crevice, which in cystalysin is built up mainly by hydrophobic residues (Leu17, Leu21, Met271, Phe273), while in MalY this region is dominated by the polar residues Glu23, Arg24, Lys261, Ser267 and Ser268. (2) MalY contains a binding patch for the MalT protein which is the central activator of the maltose operon. The most important element of the corresponding protein-protein interaction motif of MalY, the loop comprising residues 212 to 222, is replaced by α-helix 10 (Glu224-Asp228) in cystalysin, which covers the described hydrophobic groove of the MalY interaction patch.

Neither comparison of three-dimensional structures nor amino acid sequences revealed any similarity of the hemolytically active cystalysin with known thiol-activated hemolysins of other species. Most striking is the high content of lysine residues (10.5 %) in cystalysin similarly to thiol-activated streptolysins and to α-hemolysin from *S. aureus.* The structure of staphylococcal α-hemolysin from *S*. *aureus* is characterized by a mushroom-shaped heptameric pore, that contains a lytic transmembrane domain formed by a hydrophobic β-barrel (Song et al., 1996). In cystalysin the total number of 116 charged amino acids (43 lysins, 32 glutamates, 28 aspartates and 13 arginines) out of 394, renders the surface extremely polar and opposes the formation of such a hydrophobic structure element and thus membrane insertion. Also the creation of irregular holes in erythrocyte membranes upon incubation with cystalysin (Chu et al., 1994) implies that the enzyme should function through a unique mechanism of cell lysis.

Cystalysin is highly soluble, displays remarkable stability over a wide pH-and temperature range and is almost inert to protease treatment (Chu et al., 1997). No precursor protein containing an N-terminal signal sequence has been detected as is usually required for extracellular translocation of periplasmic, outer membrane, and secreted proteins (Oliver, 1985). Also the multiple motif LXGGXGXD, previously described for proteins secreted via alternate pathways (Delepelaire and Wandersman, 1990) is absent in cystalysin. Moreover, all PLP-enzymes known so far are cytosolic enzymes. These findings suggest that cystalysin is not acting directly as a hemolysin but induces hemolysis and hemoxidation (Chu et al., 1994) by its reaction products (see below).

In the center of each cystalysin monomer the PLP cofactor is bound in a wide catalytic cleft. This crevice is formed by both domains of one subunit and by parts of the large domain from the neighbouring subunit (see below). The boundaries framing the active site cleft are constituted by the C-terminal tails of strands d, e and f, their connecting loops, by the N-terminal ends of helices 5 and 6 and predominantly by helix 1 building up a bordering wall throughout its length. Binding of the cofactor is achieved by Lys238 located at a β-turn connecting β-strands f and g at the bottom of the active site cleft. Continuous electron density confirms the formation of an aldimine bond between the ε-amino group of Lys238 and the aldehyde of the uncomplexed PLP (Figure 2A). Additionally, the cofactor is anchored strongly through its phosphate group which has a total of six hydrogen bonds with protein residues (Ser237, Val98, Val99 and Tyr64*) and two hydrogen bonds with two water molecules (Figure 2B). Further polar interactions are found between Asp203 and the pyridine N1 as well as between His206 and Asn175 and the deprotonated hydroxyl group 03'. Furthermore the cofactor displays ring stacking interactions with the phenolring of Tyr123, that is positioned 3.8 Å above the pyridine ring in parallel orientation. Ring-stacking interactions and stabilization of the positively charged pyridine nitrogen are conserved features in the α-family of PLP-enzymes and have been shown to contribute to the electron sink character of the conjugated pyridine system (Yano et al., 1992).

The superpositions of cystalysin with MalY was performed with the program O (Jones et al., 1991) and revealed similar active site architectures (Figure 2C). Most of the active site residues of MalY are conserved in cystalysin including Asn175, Asp203, Lys238 (Schiff base), Arg369, Tyr64*, Asp40, Val98, Tyr123, Cys171, Pro173, His206 and Ser237. Only in cystalysin, Tyr124 occupies a unique position, reaching towards Tyr123 into the active site and thus limiting substrate approach of PLP cofactor above the pyridine ring area. Most probably, Tyr124 is crucial to determine the substrate preference of cystalysin and may act in concert with Phe162, Phe360 and Ile359 which also restrict the active site accessibility in this direction. A noticeable difference comparing the active site structures of various PLP-dependent enzymes is the pyridoxal sandwiching residue. In most aminotransferases a tryptophan or phenylalanine residue is observed in this position, while in cystathionine γ-synthase, in CBL, in tyrosine aminotransferases and in cystalysin a tyrosine is found (Metha et al., 1989). As reported previously (Clausen et al., 1996), a tyrosine in this position may be of mechanistical importance for substrate activation (see below).

Like MalY and CBL, cystalysin belongs to the group of PLP-dependent C^{β}-S^{*γ*} lyases that produce ammonium and pyruvate. A common catalytic mechanism for this class of PLP enzymes can be anticipated. Differences are solely restricted to substrate preferences: while MalY prefers cystine over the CBL main substrate cystathionine (Zdych et al., 1995) cystalysin seems to favor cysteine.

For transaldimination, the amino group of the substrate has to be deprotonated to perform a nucleophilic attack on the C4' of the internal aldimine. Substrate activation can be achieved by three ways: 1) An uncharged nitrogen of the internal aldimine can be responsible for substrate deprotonation as commonly encountered with AATs (Jansonius, 1998), 2) the prevailing pH in solution may be sufficient to shift the chemical equilibrium to the deprotonated substrate amino group (Hayashi and Kagamiyama, 1997) and 3) deprotonation is mediated by a further active site base, e.g. the phenolate oxygen of the PLP sandwiching tyrosine as proposed for the γ-family of PLP-enzymes (Clausen et al., 1996). Since the absorption maximum in the cystalysin spectrum is observed at 419 nm at pH 8.0, the aldimine nitrogen is present in its protonated state, excluding any deprotonating ability. At the pH-optimum of cystalysin between pH 7.8 and 8.0 (Chu et al., 1997) a significant amount of substrate should be present in deprotonated state to account for the maximum turnover number, since K_{cat} of 12 s⁻¹ of the overall reaction is rather slow (Chu et al., 1997). Additionally, the PLP sandwiching Tyr123 can be expected to exist at least partially as a phenolate at this pH and thus could be further supporting substrate activation in analogy with CBL.

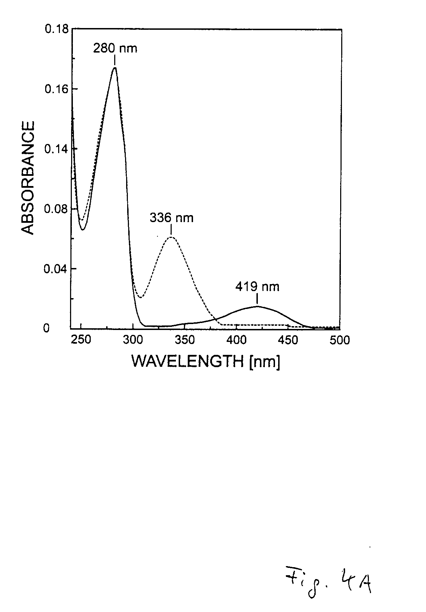

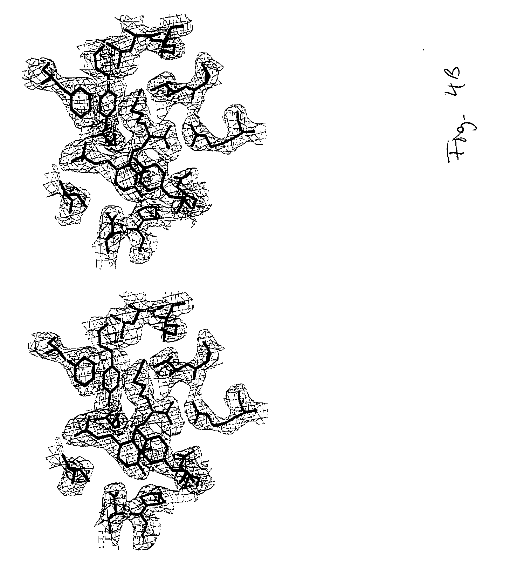

The substrate α-carboxylate should be bound by Arg369, a conserved residue in nearly all PLP-dependent enzymes that is supposed to act as a docking site for carboxylate groups (Metha et al., 1993) (see below). After transaldimination the released Lys238 abstracts the C^{α}-proton of the substrate with its deprotonated ε-amino group, producing a carbanionic intermediate that is stabilized as the characteristic quinonoid-intermediate (Figure 3). Charge dissipation into the pyridoxal system is supported by ring-stacking interactions with Tyr123 and N1-charge stabilization by Asp203. For C^{β}-S^{γ} bond cleavage the now positively charged amino group of Lys238 is attracted by the negative charge of the PLP-phosphate group and further guided towards S^{γ} by interaction with Tyr64*, the carbonyl oxygen of Ala39 and one well-defined active site water. After reaching an optimal position within hydrogen bonding distance to S^{γ}, Lys238 protonates the sulfur atom. The resulting thiol is released, which can be H₂S (cysteine as a substrate), homocysteine (cystathionine as a substrate) or cysteine persulfide (cystine as a substrate). Finally, reverse transaldimination of the PLP aminoacrylate takes place. Lys238 attacks the C4' atom, converting the aminoacrylate into an iminopropionate derivative. The reaction end product iminopropionate is released and hydrolyzed to pyruvate and ammonia outside the active site.

Altogether, CBL, MalY and cystalysin are catalyzing a C-S lyase reaction with basically the same organization of the active site key residues Tyr64*, Tyr123, Lys238 (CBL: Tyr56*, Tyr111, Lys210; MalY: Tyr61*, Tyr121, Lys233). However, the active site construction of these PLP enzymes results in individual substrate preferences while conservation of a similar reaction mechanism is the consequence of convergent evolution.

As is evident from the present structure, cystalysin is not a typical pore-forming protein like the classical hemolysins. Obviously, the oral pathogen *Treponema denticola* possesses a PLP-dependent enzyme, active as an L-cysteine C^{β}-S^{γ} lyase, to generate a toxic substance: H₂S. Moxness et al. (Moxness et al. 1996) proved recently that H₂S has the ability to induce cell lysis. The toxic action of H₂S is based on two effects, which may occur in combination: 1) Treatment of erythrocytes shows a depletion of phospholipids in the inner membrane leaflet relative to the outer leaflet (Kurzban et al., 1999). This causes an outwardly bulging of the membrane resulting in spikes and protrusions. The morphological change transforms the normally disc-shaped erythrocytes into ballon-like echinocytes. 2) H₂S is able to increase the permeability of oral mucosal tissue (Ng and Tonzetich, 1984) and assumingly also erythrocyte membranes. An osmotic intrusion of water molecules is the most likely effect that should finally lead to disintegration of the membrane by gaps and holes. Such a scenario would explain the irregularity and variability in size of the membrane lesions observed upon incubation with cystalysin (Chu et al., 1994). Furthermore, the cell adhesion and agglutinating properties of *T. denticola* could even enhance hemolysis. Generating elevated concentrations of H₂S that can diffuse directly through the bacterial to the erythrocyte membrane the bacteria have immediate access to the leaking erythrocyte plasma upon hemolysis. Producing H₂S, cystalysin is playing the key role in the mechanism of hemolysis as performed by *T. denticola.*

Interestingly, also the causative agent of upper respiratory tract disease, *Bordetella avium,* produces a PLP-dependent β-cystathionase acting as a cytotoxic protein (osteotoxin) (Gentry-Weeks et al., 1993). In osteogenic, osteosarcoma and tracheal cells it causes the formation of ballon-like structures and surfacial blebs that lead finally to plasmolysis and cell death. Similar to cystalysin this β-cystathionase is specific for L-cystathionine, L-cystine and L-cysteine-derivatives showing only weak affinity for L-cysteine (Kₘ = 5 mM). The toxicity of this protein was attributed to the cleavage of L-cysteine, for which osteotoxin shows the highest affinity (Kₘ = 77 µM). Thompson and coworkers (Gentry-Weeks et al., 1993) propose a reaction mechanism that could also be valid for cystalysin: sulfane sulfur derivatives containing at least two covalently linked sulfur atoms like persulfide (-SSH), trisulfide (-SSSH), elemental sulfur (S₈) and polysulfur (Sₙ) are supposed to be the chemical basis for the observed biological effects (Toohey, 1989). Sulfane sulfur products like thiocysteine and elementary sulfur are inherently unstable and have been proven to undergo rapid sulfurylation reactions with thiophilic acceptors (Gentry-Weeks et al., 1993). Thereby di- and trisulfides are formed and thiol groups in proteins are oxidated, resulting in an intermolecular transfer of sulfur atoms. The observed effects on enzyme functions can be activatory or inhibitory (Toohey, 1989). At worst, transfer of sulfane sulfur to metabolically or structurally important proteins can lead to cell death. The production of the persulfide thiocysteine and elemental sulfur could be confirmed for osteotoxin and was also observed for cystalysin. Although L-cysteine is the proposed main natural substrate, there is evidence that also cleavage of L-cystine is dramatically enhancing the hemoxidating and hemolytic activity of cystalysin (Chu et al., 1997). The incorporation of sulfur from the cleavage of L-[³⁵S]cystine could be proven for osteotoxin and resulted in labeling of the enzyme itself and other cell proteins (Gentry-Weeks et al., 1993). The effects of reactive sulfane sulfur derivatives like thiocysteine on erythrocytes have been studied by Valentine and coworkers (Valentine et al., 1987). Upon incubation rapid inhibition of the erythrocyte metabolism and vacuole formation was observed, mainly caused by inhibition of many glycolytic enzymes, that appeared to be extremely sensitive to sulfane sulfur. Hence, the reaction products resulting from cleavage of L-cystine by cystalysin could be an additional important factor in the complex process of hemolysis.

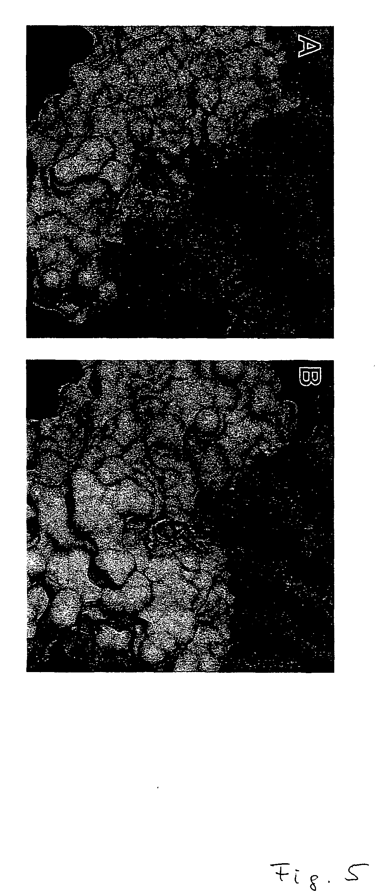

PLP-dependent enzymes catalyzing β-elimination reactions are known to be inhibited by a number of β,*γ*-unsaturated amino acids like the natural toxins rhizobitoxin (Owens et al., 1968) and L-aminoethoxyvinylglycine (AVG) (Pruess et al., 1974), that act as so-called slow-binding inhibitors (Morrison and Walsh, 1988). Interactions studies of CBL with AVG (Clausen et al., 1977) revealed the time-dependent formation of a long-lived, slowly dissociating enzyme-inhibitor compolex upon treatment with AVG. Spectral and fluorescence measurements lead to similar observations for cystalysin after incubation with AVG (Figure 4A). Treatment of cystalysin with AVG resulted in a rapid and complete loss of the pyridoxaldimine absorbance of the active enzyme at 419 nm while simultaneously a chromophore at 336 nm was formed. Crystallographic analysis of cystalysin crystals incubated with AVG showed clear and continuous electron density for a covalent PLP-AVG adduct (Figure 4B). The inhibitor is mainly bound by hydrogen bonds to its carboxylate oxygens which originate from Ala39, Asn 175 and Arg369 (Figure 4C). Interestingly, binding of the terminal amino group occurs only over a single hydrogen bond with a well-defined water molecule. Hydrophobic interactions are with Tyr64* and Phe273* which are in van-der-Waals contact to the carbon atoms C_{FI}, C_{EI} and C_{GI}. Contrary for CBL, the amino-tail of the inhibitor is not bound to Tyr123, but stretched out in opposite direction towards a large hydrophobic cavity formed by the residues Leu17, Val38, Typ64* and Phe273* that is absent in CBL. Comparison of the substrate binding pockets indicates that cystalysin displays a remarkably larger active site cleft (ca. 32 Å x 19 Å x 13 Å) than CBL (ca. 17 Å x 13 Å x 10 Å) (Figure 5). The generous construction of the active site cleft implies high accessibility to the PLP cofactor and could grant access even for bulky substrates or inhibitors. Therefore, the design of selective inhibitors, that are able to discriminate between the active sites of cystalysin and CBL, is also feasible.

Overall, the PLP cofactor remains fixed in position with only the pyridine ring rotated slightly (≤ 10°), while no major structural changes regarding domain movement have been observed. These findings are contradicting an open/closed mechanism upon substrate binding, since the cystalysin-AVG structure should resemble the physiological enzyme-substrate complex. Due to the similarities in inhibition pattern and spectral changes, AVG should act on cystalysin in a similar manner as reported for CBL (Clausen et al., 1997). After transaldimination, a proton transfer from C^{α} to C4' is mediated by the deprotonated ε-amino group of the released Lys238, instead of protonating the leaving group. The resulting "dead end" ketimine PLP derivative is responsible for the observed 336 nm-chromophore and the total loss of enzyme activity. The inability of cystalysin to catalyze a transamination of this ketimine also accounts for the high stability of the final intermediate.

The virulence potential of *T. denticola* is strongly dependent on the catabolism of thiol-substrates to reactive hydrogen sulfide and sulfane sulfur. Thus, inhibition of cystalysin should result in complete loss of hemyolytic activity (Kurzban et al., 1999). In vivo, cystalysin inhibition is likely to cause significant shortage of nutrients, especially in terms of heme as the proposed major iron source of the bacteria, resulting in limited growth and probable cell death. Secondly, missing selective advantages created by elevated H₂S concentrations in the periodontal ecological niche may stop *T. denticola* growth. With increasing resistance of bacteria to common antibiotics cystalysin appears to be an interesting novel target.

The resolution of the crystal structure of cystalysin and the cystalysin-AVG complex for the first time opens the possibility for a rational design of therapeutic agents against the oral pathogen *T. denticola.* A further subject matter of the present invention therefore is the use of the crystalline preparation of cystalysin and/or the cystalysin-AVG complex to obtain crystal structure data for the design and/or identification of inhibitors of cystalysin.

Yet another subject matter of the invention is the use of obtained crystal structure data for the design of inhibitor molecules. The design of inhibitor molecules may follow closely inhibitor AVG, but, however, may also vary therefrom to a large extent as long as the above described interactions between cystalysin and its substrate or its cofactor, or between cystalysin monomers are inhibited. From the above detailed disclosure of the crystal structure of cystalysin monomers and dimers as well as the cystalysin-AVG complex it can be recognized, which amino acids of the cystalysin molecule take part in the corresponding interactions. Therefrom it can be concluded which and how many amino acids are involved in binding to an inhibitor molecule in order to obtain the desired inhibition. By providing crystal structure data as indicated in detail above, it is possible to determine the topology of the active site and use it so that corresponding active inhibitor molecules can be postulated and produced.

In a preferred embodiment of the use according to the invention a computer-aided modelling program is used for the design of such inhibitor molecules. Such programs are known to the man in the art and are commercially available. Using the crystal structure data, it is also possible to screen molecules of interest for the presence of domains that specifically interact with cystalysin, causing an inhibition of the catalytic activity thereby, either by blocking the active site to substrate molecules or by abolishing the binding of cofactor or by inhibiting dimer formation of cystalysin monomers. Most easily also this screening is conducted using computer alignment software, which is readily available to the man in the art.

A further subject of the present invention are inhibitor molecules that are able to interact with cystalysin in such a way that the hemolytic and hemoxydizing activity of the protein is at least substantially impaired.

In a preferred embodiment of the invention the inhibitor molecule interacts with and/or binds to one or more of Leu17, Val38, Ala39, Asp40, Tyr64*, Val98, Val99, Tyr123, Tyr124, Phe162, Cys171, Pro173, Asn175, Asp203, His206, Ser237, Lys238, Phe273*, Ile356, Phe360 and/or Arg369.

In one aspect, the inhibitor molecule according to the invention is designed to mimic the natural substrate and contain a group that becomes immersed into the active site thereby interacting with the amino acids at the active site in such a way that a competitive binding takes place between natural substrate and inhibitor. Depending on further interactions between inhibitor and cystalysin in comparison to the natural substrate, a preferential binding of inhibitor can be achieved.

It is, however, also conceivable, that the inhibitor molecule is designed in such a way that the molecule is unable to form a dimer and/or to bind a PLP cofactor. In this respect, it is not absolutely necessary that the inhibitor molecule binds to the specific amino acids at the relevant positions, an inhibitor molecule could also bind to and/or interact with groups neighboring the active site, dimer binding or cofactor binding domain as long as it is "bulky enough" to more or less shield the specific amino acids so that the natural substrate and/or the cofactor cannot bind, or the monomers cannot form dimers anymore.

An inhibitor molecule according to the invention preferably is designed to be able to bind to several of the above described specific amino acids covalently or non-covalently. Hydrophobic groups or groups interacting via salt bridges and hydrogen bonds can also be present.

According to one embodiment of the invention, therefore, the inhibitor molecule binds covalently to cystalysin. Such covalent binding occuring either reversibly or irreversibly, is very efficient in inhibiting the activity of cystalysin. Irreversible bonds can e.g. be formed by nitriles (formation of isothiamid), halomethyl ketones, diazomethyl ketones, acyloxymethyl ketones, methylsulfonium salts, epoxysuccinyl derivatives, vinyl sulfones, O-acylhydroxamates, aciridines or activated disulfides. Formation of covalent hydrolytically labile bonds between an inhibitor and cystalysin leads to reversible inhibition and can be accomplished by using e.g. aldehydes with thioactetal formation, methyl ketones and trifluoromethyl ketones under formation of tetrahydryl hemiketals or α-keto acids, -esters or -amids or -diketones.

It is, however, equally possible that the inhibitor molecule is designed to non-covalently bind to cystalysin. Such non-covalent binding can be effected through reactive groups that are capable of forming salt bridges or hydrogen bonds with the specific amino acids of cystalysin as described above or molecules in their neighborhood.

The inhibitor molecule according to the invention is preferably designed to be able to interact with several anchor groups and therefor has to have a structure that allows for such interactions in view of length and breadth. For this purpose the inhibitor preferably contains a backbone chain, to which one or more specific reactive groups are attached that mediate the binding and/or interaction. Additionally, the backbone chain itself can interact with the relevant sites and their environments, for example via hydrogen bonds thereby further strengthening the binding/interaction.

Such backbone chain can be of any nature as long as it is possible that the reactive groups arrange in a specifically oriented manner. The backbone chain is preferably a peptide or a peptidomimetic chain, but can also be any other organic molecule. A polypeptidic or peptidomimetic backbone chain has the advantage that it can be formed for example close to the natural substrate, however, also much shorter, containing reactive groups that bind stronger or to more anchor groups at the relevant sites of cystalysin.

Organic molecules can also be favorably present as backbone chain, for example in view of cost, ease of production, versatility for attaching reactive groups thereto etc.

In one embodiment of the invention, the cystalysin inhibitor contains a polypeptide chain backbone with 1 to 10 amino acids and more preferred 2 to 8 amino acids, or a synthetic organic backbone chain that has a length corresponding to such polypeptide chain length.

If a non peptidic organic backbone chain or a combination of peptidic and non-peptidic residues is present, it is preferred that the flexibility of the chain is reduced to decrease the entropic price for binding of the inhibitor molecule to the enzyme, but only to an extent that allows the functional groups of an inhibitor molecule to adjust to the appropriate anchor groups of cystalysin. It is therefore preferred that such chain is a hydrocarbon chain optionally containing heteroatoms, substitutions, double or triple bonds, and/or ring systems, optionally containing heteroatoms, to adjust the flexibility of the chain. As already mentioned above, the backbone chain itself can also form bonds with the cystalysin anchor groups or active site environment, thereby stabilizing the position of the specifically binding reactive groups and/or the whole inhibitor molecule.

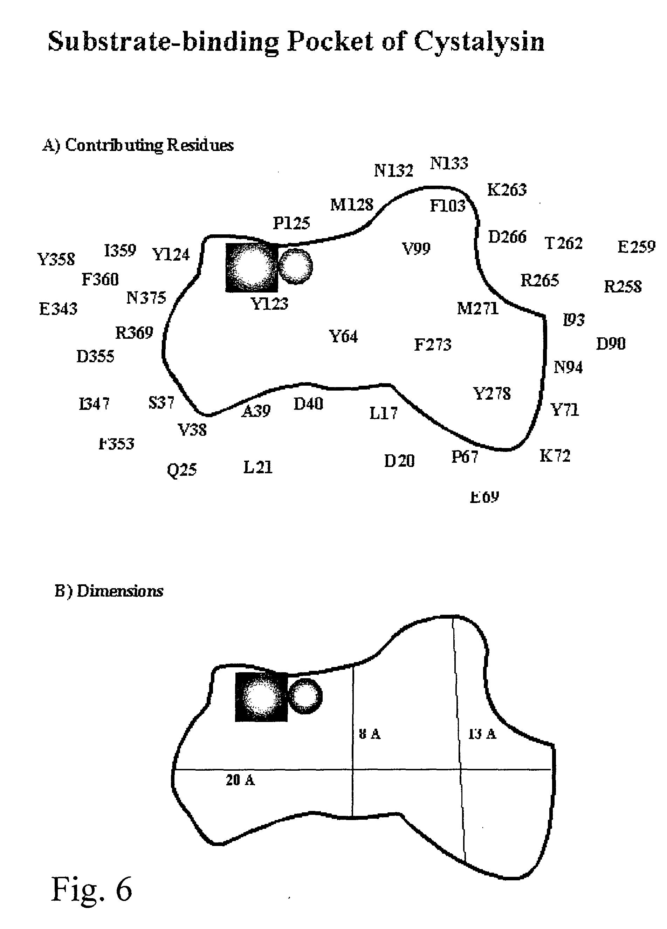

L-aminoethoxyvinylglycine can be regarded as one possible lead compound for drug design, among other possible compounds. It can be used to derive features of inhibitor molecules that (a) have affinity for the active site, (b) are not cleaved in a substrate like manner, and (c) are specific for the target enzyme cystalysin. These features are given for illustration, and are not intended to limit the scope of the present invention.
(a) As for active site affinity, the binding mode of L-aminoethoxyvinylglycine (see Fig. 4c) to cystalysin identifies at least two functional groups that are involved in anchoring the molecule to the active site. These two groups are the carboxylate and the α-amino group of L-aminoethoxyvinylglycine. The carboxylate group is hydrogen bonded to Arg 369 (see Fig. 4c). The observation that cystalysin related PLP-dependent enzymes can recruit a sulfate ion in this position shows that any negatively charged end group, in particular derivatives of sulfates, borates and phosphates can replace the carboxylate group. Additional anchoring to the active site is provided by the α-amino group of the inhibitor, that displaces the internal aldimine.
(b) A first strategy to prevent substrate-like cleavage can be derived from the reaction mechanism of cystalysin. As can be seen from the reaction mechanism, protonation of a leaving group by Lys 238 is essential for cleavage (see Fig. 2, step 5). Any inhibitor molecule that lacks the α-amino-group will fail to generate Lys 238 with a free ∈-amino group and will therefore not be cleaved. A general strategy to convert cystalysin substrates into cystalysin inhibitors is therefore the omission or substitution of the ∈-amino-group of the inhibitor, provided this is done without undue loss of affinity for the enzyme.
   A second strategy to prevent substrate-like cleavage can be inferred from the mode of inhibition of cystalysin by L-aminoethoxyvinylglycine. Its main feature is the introduction of a double bond between the β and *γ* positions in the side chain where cleavage would occur in the substrate. However, it has to be considered that the orientation of the double bond as enforced by the aminoethoxy part of the molecule has a role in the prevention of cleavage. This fact is demonstrated by the finding that methoxyvinylglycine does not inhibit the cystalysin related enzyme cystathionine β-lyase (Clausen et al., Biochemistry, **36,** 12633-12643).
(c) A particular concern of inhibitor design is always the prevention of unwanted cross reactions with human enzymes. Although this is a lesser concern for cystalysin inhibitors, that could be applied locally to the subgingiva of patients, retaining specificity of the inhibitor for cystalysin over other enzymes, in particular PLP enzymes, is still preferred. The present invention provides means to achieve this specificity. Cystalysin has a very wide active site cleft, that should grant access to bulky substrates. Specificity for cystalysin can be achieved with bulky groups that bind to the active site cleft. This distal part of this cleft can be subdivided into two smaller clefts, one above residues Val99 and Phe103, and the other above residues Met271, Phe273 and Tyr278 (see Fig. 6). Bulky groups on the inhibitor molecule, that bind to these pockets have to be 5-9 Å and 9-13 Å away from the carboxylate or related group on the inhibitor that binds to Arg 369.

In summary, the above disclosure of the crystal structure of cystalysin enables the man in the art to design and produce inhibitor molecules that by interaction or binding to cystalysin either block binding of the natural substrate and its cleavage, or block dimer formation or cofactor binding. All the embodiments of inhibitor molecules containing groups specifically binding to one or more or the described relevant sites of cystalysin and the anchor groups being present there can be designed by the man in the art using the knowledge of the nature of the anchor groups and their respective distances as shown in Fig. 1, 2, 4 and 6 and described above. Using the given data it is well within the skill of the man in the art to design inhibitor molecules that interact specifically with cystalysin. Such inhibitor molecules therefore can be advantageously used in pharmaceutical compositions which constitute a further subject of the present invention. Such pharmaceutical compositions according to the invention contain the inhibitor optionally together with pharmaceutically acceptable carrier and adjuvant substances.

The inhibitor and the optional adjuvants are used in such amounts that at least a considerable inhibition of cystalysin is achieved. The pharmaceutical compositions can be used either to prevent or to treat peridontal diseases and other illnesses that are caused or affected by primary infections with *T. denticola.* Such use of the pharmaceutical composition for the treatment and/or prevention of periodontal diseases, especially periodontitis, and/or cardiovascular diseases that might occur after a primary periodontal disease is a still further subject of the present invention.

Suitable amounts of inhibitor, necessary to achieve the desired treatment or prevention, can easily be determined by the skilled artisan. The form of application will normally be oral, an application into periodontal pockets will be most preferable. Such application can for example take place in form of a gel, a salve, impregnated fibres (like silk fibres) or any other suitable formulation that can be introduced into the periodontal pockets. Sticky formulations are preferred for a prolonged persistency of the active substance in the infected area.

Since primary periodontal diseases may lead to potentially life-threatening secondary diseases, the inhibitors according to the invention and especially the pharmaceutical compositions are important agents that can help avoid or cure the nasty periodontal diseases thereby avoiding manifestation of the more dangerous secondary diseases. The inhibitors according to the invention and pharmaceutical compositions containing such inhibitors therefore represent a considerable progress in pharmaceutics that can help avoid inter alia high cost for the treatment of periodontitis and secondary diseases, which is not adequately effective at present.

### Examples and figures further illustrate the invention:

### Examples

### Example 1

### Enzyme purification and crystallization

Cystalysin was expressed from *T. denticola* in E. coli as described previously (Chu & Holt, 1994). Shortly, E. coli DH5α transformed with plasmid pLC67 were grown in 2-liter Erlenmeyer flasks with 1 liter of Luria broth at 37°C. Induction was performed at OD₆₀₀ of 0.8 with 1 mM isopropyl-β-D-thiogalactopyranosid for up to four hours. Cells were harvested by centrifugation, resuspended with 10 ml of buffer A (1 x PBS, pH 7.2) and stored at -70°C until further processing.

The course of purification was monitored by the cystalysin cystathionase activity (Chu et al., 1997) and by SDS-PAGE. Protein concentration was determined spectroscopically at 280 mn using the extinction coefficient of 63440 M⁻¹ cm⁻¹ and the molecular weight of the cystalysin monomer of 46258 Da. After cell lysis by ultrasonic treatment and removal of cell debris by centrifugation, the enzyme was basically purified by a four-step procedure. Initially, the soluble cell fraction was submitted to an (NH₄)₂SO₄ fractionation. Solid (NH₄)₂SO₄ was added at 4°C to 60% saturation. The mixture was stirred for 20 min and the precipitate removed by centrifigation at 10 000 rpm for 30 min. (NH₄)₂SO₄ was added to the supernatant, then (NH₄)₂SO₄ was added to a final concentration of 90%. The precipitated pellet was collected by centrifugation (10 000 rpm, 30 min) and dissolved in 12 ml buffer B (25 mM Tris/HCl, pH 7.5, 1 mM EDTA). The protein solution was loaded on a Sephacryl S-300 (Pharmacia, 300 ml) gel filtration column, equilibrated with buffer B. Eluted fractions were pooled, adjusted to 1.0 M (NH₄)₂SO₄ and applied to a Phenyl Sepharose HP (Pharmacia, 80 ml) column, equilibrated with buffer B containing 1.0 M (NH₄)₂SO₄. After washing, elution was performed with buffer B in a linear decreasing salt gradient. Cystalysin containing fractions were pooled, and concentrated with centripreps (Amicon), to 40 mg/ml. The concentrated protein solution further purified by the preparative gel electrophoresis by the method of Reuther (Reuther and Wehrmeyer, 1988). The acrylamid concentration in the gel was 5%. Separation was achieved in buffer C (100 mM Tris/boric acid, pH 7.9) using a constant current of 56 mA which was applied for 3.5 h at 8°C. The protein was eluted with buffer D (10 mM Tris/HCl, pH 7.5), concentrated with centricons (Amicon) to 10 mg/ml and stored at -70°C. From 6 liters of culture 60 mg of homogeneous protein were obtained, that appeared to be 99% pure as deduced from SDS-PAGE.

Crystallization was carried out at 20°C using the sitting drop vapor diffusion method. After 2 days crystals were obtained from 10 mg/ml protein solution and 15% (w/v) PEG 4000, 0.2 M Mg-acetate and 100 mM Mes/NaOH, pH 6.5 as reservoir solution. The rhombic crystal plates of 0.6 x 0.4 x 0.1 *µ*m³ dimensions were brightly yellow, indicative of the protonated internal aldimine. They belonged to the monoclinic space group P2₁ with unit cell constants of a = 89.5 Å, b = 108.4 Å, c = 179.1 Å and β = 90.2°.

### Example 2

### Data collection, structure solution and refinement

X-ray data was collected at the wiggler beamline BW6 at DORIS (DESY, Hamburg, Germany) on a CCD-detector (MAR Research) with synchrotron radiation at 1.07 Å. A cryostream (Oxford Cryosystems, Oxford, UK) was used and the crystal was frozen at a temperature of 100 K with 0.1 M Mes/NaOH, pH 6.5, 0.2 M Mg-actetate and 20% PEG 400 as cryo-protectant. One crystal was sufficient to collect 450 images in a 90° φ-scan with an exposure time of 30 s per frame. Reflection data of native cystalysin was processed to a maximum resolution of 1.9 Å. Data of the protein-inhibitor complex was collected at a MAR image plate detector equipped with a rotating CuK_{α} anode operated at 120 mA and 50 kV. The dataset of the inhibitor complex was obtained by soaking a native cystalysin crystal for 30 min in the crystallization solution containing 20 mM AVG. Subsequently, the crystal was frozen at a temperature of 100 K (Oxford Cryosystems, Oxford, UK) and cryo-measured in 0.3° oscillation steps.

The diffraction data were processed with the DENZO program package (Otwinowski and Minor, 1996); further reduction and truncation of structure factors were performed with the CCP4 program suite (CCP4, 1994). Collection statistics for both data sets are given in Table 1. The structure of the PLP-protein MalY from E. coli (Clausen et al., 2000) was chosen as a model to solve the structure with Patterson search techniques using the AMoRe program (Navaza, 1994). In the partial model of the dimeric MalY, the PLP cofactor was omitted and all nonidentical and nonrelated residues were changed to alanine. Using data between 10.0 and 4.7 Å resolution a unique solution with a correlation coefficient of 35.5% and an R-factor of 47.9% was obtained. This solution corresponds to four dimers in the asymmetric unit, which is in good agreement with the value for the Matthews coefficient of 2.31 Å³/Da, corresponding to a solvent content of 47%.

Initial phases for the protein-inhibitor complex were obtained by molecular replacement with the refined coordinates of native cystalysin. The data processed likewise in the following:

The initial model was refined by energy restrained rigid-body and positional refinement as well as by simulated annealing using CNS (Brünger et al., 1998) and the target parameters of Engh and Huber (Engh and Huber, 1991). To improve the original 2Fₒ-F_{c} electron density map, the program AVE (Jones, 1992) was applied for 8-fold real-space averaging. The non-crystallographic symmetry operators were extracted with LSQMAN (Kleywegt, 1996). The resulting electron density map was of excellent quality and allowed the incorporation of the complete molecular structure of cystalysin. Model building was done with the program O (Jones et al., 1991) implemented on an ESV-30 graphics station (Evans and Sutherland, Salt Lake City, UT, USA) and on Silicon Graphics Indigo and Octane workstations. Crystallographic refinement during model building was carried out applying weak non-crystallographic symmetry (NCS) restraints. After the first refinement cycle the R-factor calculated 1.90 Å resolution dropped from 44.4% (R_{free} 45.6%) to 28.4% (R_{free} 31.7%). In later stages positional and B-factor refinement were carried out omitting NCS restraints. After the additon of solvent molecules the refinement converged at a crystallographic R-factor of 20.8% (R_{free} 24.7%).

### Figure 1

### Overall fold

(A) Structure of the cystalysin monomer showing the large domain with green helices and magenta β-sheets, the small domain with blue helices and the PLP cofactor in yellow. The nomenclature for the secondary structural elements is indicated. (B) Ribbon presentation of the cystalysin dimer emphasizing structural elements essential for dimerization. Helical structures are held in green, loop structure in blue and β-sheets in magenta. 90°-rotation results in the characteristic S-shaped orientation in which the sequential flow is mapped by color ramp to one monomer (N-terminus: blue; C-terminus: red).

### Figure 2

### Active site structue

(A) Final 2Fₒ-F_{c} electron density of the PLP cofactor (yellow) and the immediate protein vicinity (color-coding by atom type), contoured at 1.2 σ and calculated at 1.9 Å resolution. (B) Schematic presentation of the active site around the PLP cofactor (red). Residues contributing from the neighboring subunit are colored green. Hydrogen bonds and interatomic distances (Å) are indicated. (C) Stereo plot showing the superposition of the active sites of cystalysin and MalY from E. coli. Cystalysin residues are colored grey with the cofactor in yellow. MalY is shown in blue and its PLP in orange.

### Figure 3

### Proposed molecular reaction mechanism for cystalysin showing relevant intermediates

The PLP cofactor is colored black, the substrate and leaving product in red and the enzyme residues in blue.

### Figure 4

### The cystalysin-AVG complex

(A) The spectra of uncomplexed cystalysin and cystalysin after treatment with AVG, recorded at 20°C in 100 mM phosphate buffer. (B) Final 2Fₒ-F_{c} electron density of the PLP-AVG complex (yellow) and the intermediate protein vicinity (color-coding by atom type), contoured at 1.2 σ and calculated at 2.5 Å resolution. (C) Schematic presentation of the PLP-AVG complex (red) depicted with polar and hydrophobic interactions. Interatomic distances are given in Å.

### Figure 5

### Active site morphology

Surface plots of the active site cavities of cystalysin (A) and CBL (B) both with the PLP-AVG complex in yellow (color coding by atom type, P green). The surface of the subunit, harboring the complex is colored in grey, the neighboring subunit in orange.

### Figure 6A

### Schematic representation of the cystalysin active site pocket

Schematic representation of residues in the vicinity of the active site. The PLP cofacter is indicated in symbolic representation. Residues that form the bottom of the pocket are drawn within the marked area. The other residues form the active site walls. The large pocket can be devided into two minor ones, one above residues Phe103 and Va199, the other above residues Met271, Phe273 and Tyr278.

### Figure 6B

### Diagram of the distances of the cystalysin active site pocket

**Table 1**

| Statistics for data collection and refinement | | |
|---|---|---|
| | Cystalysin | Cystalysin-AVG inhibitor-complex |
| Space group | P2₁ | P2₁ |
| Cell constants | a = 89.47 Å, | a = 89.37 Å, |
| | b = 108,44 Å, | b = 107.93 Å, |
| | c = 176.09 Å, | c = 176.16 Å, |
| | β = 90.17° | β = 90.17° |
| Resolution range (Å) | 25 - 1.9 | 20 - 2.5 |
| Reflections observed | 382434 | 149605 |
| Unique reflections | 254956 | 106861 |
| R_{merge}^{a} overall (%) | 7.8 | 4.6 |
| R_{merge}^{a} outermost shell (%) | 25.5 (1.97 - 1.90 Å) | 20.4 (2.59 - 2.50 Å) |
| Completeness overall (%) | 96.5 | 92.4 |
| Completeness outermost shell (%) | 87.9 (1.97 - 1.90 Å) | 87.0 (2.59 - 2.50 Å) |
| I/σ overall (I) | 8.6 | 28.9 |
| Reflections used for refinement | 254953 | 106861 |
| Active protein atoms | 25664 | 25664 |
| Active cofactor atoms | 120 | 120 |
| Inhibitor atoms | - | 88 |
| Solvent molecules | 2627 | 1300 |
| R value^{b} final model (%) | 20.8 | 19.3 |
| R_{free} value^{c} final model (%) | 24.7 | 26.0 |
| Rms deviations | | |
| - bond lengths (Å) | 0.006 | 0.007 |
| - bond angles (°) | 1.4 | 1.4 |
| - dihedral angles (°) | 22.5 | 22.6 |
| - improper angles (°) | 0.87 | 0.85 |
| Temperature factors (Å²) | | |
| - all atoms | 16.9 | 30.4 |
| - protein main chain atoms | 16.0 | 29.9 |
| - protein side chain atoms | 17.8 | 30.9 |
| - cofactor (and inhibitor) atoms | 20.6 | 39.8 |
| - solvent molecules | 39.3 | 43.0 |

| | | |
|---|---|---|
| ^{a}R_{merge} = Σ(I-<I>)/ΣI | | |
| ^{b}R = Σ(IF_{obs}I-IF_{calc}I)/ΣIF_{obs}I | | |
| ^{c}R_{free} was calculated with 5% of the observed reflections which were omitted from the refinement and R-value calculation. | | |

**Table 2**

| Cystalysin: Coordinates of residues in the vicinity of the active site | | | | | | | | |
|---|---|---|---|---|---|---|---|---|
| ATOM | 131 | N | LEU 17 | 17.896 | -14.491 | 16.455 | 1.00 | 23.10 |
| ATOM | 132 | CA | LEU 17 | 18.733 | -14.105 | 17.581 | 1.00 | 23.61 |
| ATOM | 133 | CB | LEU 17 | 18.741 | -12.583 | 17.756 | 1.00 | 23.74 |
| ATOM | 134 | CG | LEU 17 | 19.711 | -11.886 | 16.787 | 1.00 | 24.96 |
| ATOM | 135 | CD1 | LEU 17 | 19.177 | -11.930 | 15.368 | 1.00 | 27.55 |
| ATOM | 136 | CD2 | LEU 17 | 19.909 | -10.458 | 17.202 | 1.00 | 27.10 |
| ATOM | 137 | C | LEU 17 | 18.277 | -14.806 | 18.856 | 1.00 | 21.52 |
| ATOM | 138 | O | LEU 17 | 19.090 | -15.141 | 19.715 | 1.00 | 22.55 |
| ATOM | 162 | N | ASP 20 | 19.226 | -18.808 | 18.417 | 1.00 | 24.33 |
| ATOM | 163 | CA | ASP 20 | 20.688 | -18.910 | 18.427 | 1.00 | 25.13 |
| ATOM | 164 | CB | ASP 20 | 21.311 | -17.960 | 17.398 | 1.00 | 25.06 |
| ATOM | 165 | CG | ASP 20 | 21.092 | -18.415 | 15.973 | 1.00 | 27.46 |
| ATOM | 166 | OD1 | ASP 20 | 20.408 | -19.445 | 15.763 | 1.00 | 28.13 |
| ATOM | 167 | OD2 | ASP 20 | 21.608 | -17.732 | 15.060 | 1.00 | 27.48 |
| ATOM | 168 | C | ASP 20 | 21.260 | -18.600 | 19.803 | 1.00 | 23.82 |
| ATOM | 169 | O | ASP 20 | 22.237 | -19.216 | 20.234 | 1.00 | 22.14 |
| ATOM | 170 | N | LEU 21 | 20.655 | -17.632 | 20.484 | 1.00 | 25.91 |
| ATOM | 171 | CA | LEU 21 | 21.101 | -17.252 | 21.819 | 1.00 | 27.10 |
| ATOM | 172 | CB | LEU 21 | 20.307 | -16.053 | 22.326 | 1.00 | 27.77 |
| ATOM | 173 | CG | LEU 21 | 20.662 | -15.640 | 23.757 | 1.00 | 31.57 |
| ATOM | 174 | CD1 | LEU 21 | 22.162 | -15.338 | 23.846 | 1.00 | 31.67 |
| ATOM | 175 | CD2 | LEU 21 | 19.834 | -14.425 | 24.166 | 1.00 | 31.04 |
| ATOM | 176 | C | LEU 21 | 20.913 | -18.428 | 22.775 | 1.00 | 26.09 |
| ATOM | 177 | O | LEU 21 | 21.759 | -18.686 | 23.632 | 1.00 | 25.67 |
| ATOM | 204 | N | GLN 25 | 23.748 | -20.316 | 25.033 | 1.00 | 33.23 |
| ATOM | 205 | CA | GLN 25 | 23.761 | -20.492 | 26.482 | 1.00 | 32.76 |
| ATOM | 206 | CB | GLN 25 | 22.604 | -19.724 | 27.113 | 1.00 | 31.49 |
| ATOM | 207 | CG | GLN 25 | 22.848 | -18.242 | 27.248 | 1.00 | 31.97 |
| ATOM | 208 | CD | GLN 25 | 21.557 | -17.453 | 27.259 | 1.00 | 33.53 |
| ATOM | 209 | OE1 | GLN 25 | 21.543 | -16.281 | 27.629 | 1.00 | 34.86 |
| ATOM | 210 | NE2 | GLN 25 | 20.459 | -18.091 | 26.841 | 1.00 | 33.10 |
| ATOM | 211 | C | GLN 25 | 23.693 | -21.951 | 26.931 | 1.00 | 32.74 |
| ATOM | 212 | O | GLN 25 | 24.349 | -22.329 | 27.900 | 1.00 | 31.71 |
| ATOM | 294 | N | SER 37 | 12.344 | -15.964 | 25.558 | 1.00 | 21.32 |
| ATOM | 295 | CA | SER 37 | 13.010 | -14.726 | 25.963 | 1.00 | 21.52 |
| ATOM | 296 | CB | SER 37 | 14.520 | -14.956 | 26.009 | 1.00 | 20.45 |
| ATOM | 297 | OG | SER 37 | 14.996 | -15.448 | 24.768 | 1.00 | 21.97 |
| ATOM | 298 | C | SER 37 | 12.701 | -13.536 | 25.046 | 1.00 | 21.86 |
| ATOM | 299 | O | SER 37 | 11.676 | -12.873 | 25.200 | 1.00 | 22.10 |
| ATOM | 300 | N | VAL 38 | 13.599 | -13.277 | 24.100 | 1.00 | 21.76 |
| ATOM | 301 | CA | VAL 38 | 13.471 | -12.184 | 23.136 | 1.00 | 20.68 |
| ATOM | 302 | CB | VAL 38 | 14.327 | -12.473 | 21.885 | 1.00 | 23.00 |
| ATOM | 303 | CG1 | VAL 38 | 14.181 | -11.343 | 20.867 | 1.00 | 22.70 |
| ATOM | 304 | CG2 | VAL 38 | 15.790 | -12.628 | 22.291 | 1.00 | 21.28 |
| ATOM | 305 | C | VAL 38 | 12.043 | -11.877 | 22.688 | 1.00 | 19.59 |
| ATOM | 306 | O | VAL 38 | 11.249 | -12.776 | 22.440 | 1.00 | 19.65 |
| ATOM | 307 | N | ALA 39 | 11.725 | -10.593 | 22.565 | 1.00 | 20.18 |
| ATOM | 308 | CA | ALA 39 | 10.384 | -10.182 | 22.157 | 1.00 | 22.41 |
| ATOM | 309 | CB | ALA 39 | 10.073 | -8.795 | 22.714 | 1.00 | 22.79 |
| ATOM | 310 | C | ALA 39 | 10.009 | -10.218 | 20.667 | 1.00 | 23.23 |
| ATOM | 311 | O | ALA 39 | 9.782 | -9.167 | 20.056 | 1.00 | 23.98 |
| ATOM | 312 | N | ASP 40 | 9.977 | -11.411 | 20.079 | 1.00 | 22.17 |
| ATOM | 313 | CA | ASP 40 | 9.506 | -11.576 | 18.704 | 1.00 | 24.54 |
| ATOM | 314 | CB | ASP 40 | 10.652 | -11.761 | 17.671 | 1.00 | 28.20 |
| ATOM | 315 | CG | ASP 40 | 11.406 | -13.081 | 17.805 | 1.00 | 29.57 |
| ATOM | 316 | OD1 | ASP 40 | 12.361 | -13.275 | 17.012 | 1.00 | 29.57 |
| ATOM | 317 | OD2 | ASP 40 | 11.066 | -13.910 | 18.672 | 1.00 | 30.68 |
| ATOM | 318 | C | ASP 40 | 8.589 | -12.789 | 18.840 | 1.00 | 25.03 |
| ATOM | 319 | O | ASP 40 | 8.712 | -13.543 | 19.810 | 1.00 | 26.66 |
| ATOM | 506 | N | TYR 64 | 16.758 | 1.920 | -4.473 | 1.00 | 18.63 |
| ATOM | 507 | CA | TYR 64 | 16.574 | 2.938 | -3.436 | 1.00 | 19.86 |
| ATOM | 508 | CB | TYR 64 | 17.453 | 4.158 | -3.731 | 1.00 | 19.30 |
| ATOM | 509 | CG | TYR 64 | 18.921 | 3.889 | -3.491 | 1.00 | 19.75 |
| ATOM | 510 | CD1 | TYR 64 | 19.408 | 3.693 | -2.200 | 1.00 | 19.87 |
| ATOM | 511 | CE1 | TYR 64 | 20.747 | 3.359 | -1.975 | 1.00 | 19.78 |
| ATOM | 512 | CD2 | TYR 64 | 19.810 | 3.750 | -4.557 | 1.00 | 18.90 |
| ATOM | 513 | CE2 | TYR 64 | 21.146 | 3.410 | -4.344 | 1.00 | 18.97 |
| ATOM | 514 | CZ | TYR 64 | 21.608 | 3.213 | -3.051 | 1.00 | 18.62 |
| ATOM | 515 | OH | TYR 64 | 22.921 | 2.842 | -2.837 | 1.00 | 16.21 |
| ATOM | 516 | C | TYR 64 | 15.108 | 3.341 | -3.325 | 1.00 | 19.45 |
| ATOM | 517 | O | TYR 64 | 14.557 | 3.965 | -4.228 | 1.00 | 19.64 |
| ATOM | 529 | N | PRO 67 | 9.244 | 6.237 | 1.437 | 1.00 | 22.71 |
| ATOM | 530 | CD | PRO 67 | 9.870 | 7.129 | 2.433 | 1.00 | 20.59 |
| ATOM | 531 | CA | PRO 67 | 7.800 | 6.125 | 1.684 | 1.00 | 22.19 |
| ATOM | 532 | CB | PRO 67 | 7.658 | 6.710 | 3.078 | 1.00 | 20.29 |
| ATOM | 533 | CG | PRO 67 | 8.673 | 7.810 | 3.052 | 1.00 | 20.88 |
| ATOM | 534 | C | PRO 67 | 6.914 | 6.860 | 0.677 | 1.00 | 23.68 |
| ATOM | 535 | O | PRO 67 | 7.195 | 8.000 | 0.313 | 1.00 | 25.09 |
| ATOM | 543 | N | GLU 69 | 3.066 | 8.424 | -0.615 | 1.00 | 27.70 |
| ATOM | 544 | CA | GLU 69 | 2.022 | 9.196 | 0.044 | 1.00 | 28.12 |
| ATOM | 545 | CB | GLU 69 | 1.411 | 10.210 | -0.922 | 1.00 | 30.01 |
| ATOM | 546 | CG | GLU 69 | 2.310 | 11.411 | -1.163 | 1.00 | 35.11 |
| ATOM | 547 | CD | GLU 69 | 2.756 | 12.060 | 0.140 | 1.00 | 38.75 |
| ATOM | 548 | OE1 | GLU 69 | 1.877 | 12.537 | 0.893 | 1.00 | 41.54 |
| ATOM | 549 | OE2 | GLU 69 | 3.980 | 12.086 | 0.413 | 1.00 | 39.99 |
| ATOM | 550 | C | GLU 69 | 0.954 | 8.261 | 0.579 | 1.00 | 27.09 |
| ATOM | 551 | O | GLU 69 | 0.379 | 8.499 | 1.640 | 1.00 | 28.39 |
| ATOM | 561 | N | TYR 71 | 1.518 | 5.367 | 1.615 | 1.00 | 23.55 |
| ATOM | 562 | CA | TYR 71 | 2.140 | 4.754 | 2.777 | 1.00 | 22.35 |
| ATOM | 563 | CB | TYR 71 | 3.658 | 4.645 | 2.586 | 1.00 | 19.63 |
| ATOM | 564 | CG | TYR 71 | 4.374 | 4.245 | 3.859 | 1.00 | 19.07 |
| ATOM | 565 | CD1 | TYR 71 | 4.561 | 2.900 | 4.197 | 1.00 | 18.53 |
| ATOM | 566 | CE1 | TYR 71 | 5.127 | 2.537 | 5.416 | 1.00 | 16.85 |
| ATOM | 567 | CD2 | TYR 71 | 4.777 | 5.212 | 4.773 | 1.00 | 17.63 |
| ATOM | 568 | CE2 | TYR 71 | 5.339 | 4.864 | 5.992 | 1.00 | 18.97 |
| ATOM | 569 | CZ | TYR 71 | 5.512 | 3.533 | 6.312 | 1.00 | 18.58 |
| ATOM | 570 | OH | TYR 71 | 6.061 | 3.224 | 7.534 | 1.00 | 18.96 |
| ATOM | 571 | C | TYR 71 | 1.851 | 5.594 | 4.015 | 1.00 | 23.90 |
| ATOM | 572 | O | TYR 71 | 1.431 | 5.071 | 5.050 | 1.00 | 23.65 |
| ATOM | 573 | N | LYS 72 | 2.086 | 6.897 | 3.897 | 1.00 | 25.55 |
| ATOM | 574 | CA | LYS 72 | 1.877 | 7.827 | 4.995 | 1.00 | 27.45 |
| ATOM | 575 | CB | LYS 72 | 2.382 | 9.209 | 4.603 | 1.00 | 27.92 |
| ATOM | 576 | CG | LYS 72 | 3.887 | 9.226 | 4.377 | 1.00 | 31.03 |
| ATOM | 577 | CD | LYS 72 | 4.404 | 10.616 | 4.065 | 1.00 | 31.35 |
| ATOM | 578 | CE | LYS 72 | 5.868 | 10.569 | 3.681 | 1.00 | 33.03 |
| ATOM | 579 | NZ | LYS 72 | 6.385 | 11.928 | 3.414 | 1.00 | 36.04 |
| ATOM | 580 | C | LYS 72 | 0.427 | 7.897 | 5.434 | 1.00 | 28.15 |
| ATOM | 581 | O | LYS 72 | 0.133 | 7.789 | 6.626 | 1.00 | 28.96 |
| ATOM | 738 | N | ASP 90 | 3.365 | 14.561 | 7.528 | 1.00 | 31.54 |
| ATOM | 739 | CA | ASP 90 | 4.727 | 15.052 | 7.607 | 1.00 | 30.58 |
| ATOM | 740 | CB | ASP 90 | 4.820 | 16.459 | 7.020 | 1.00 | 33.80 |
| ATOM | 741 | CG | ASP 90 | 3.913 | 17.428 | 7.698 | 1.00 | 35.99 |
| ATOM | 742 | OD1 | ASP 90 | 3.771 | 18.559 | 7.181 | 1.00 | 37.74 |
| ATOM | 743 | OD2 | ASP 90 | 3.345 | 17.059 | 8.748 | 1.00 | 39.41 |
| ATOM | 744 | C | ASP 90 | 5.322 | 15.001 | 9.004 | 1.00 | 30.35 |
| ATOM | 745 | O | ASP 90 | 6.448 | 15.439 | 9.218 | 1.00 | 31.40 |
| ATOM | 768 | N | ILE 93 | 8.588 | 10.354 | 10.545 | 1.00 | 21.93 |
| ATOM | 769 | CA | ILE 93 | 9.995 | 10.236 | 10.223 | 1.00 | 22.16 |
| ATOM | 770 | CB | ILE 93 | 10.863 | 11.060 | 11.185 | 1.00 | 22.01 |
| ATOM | 771 | CG2 | ILE 93 | 12.331 | 10.793 | 10.905 | 1.00 | 20.56 |
| ATOM | 772 | CG1 | ILE 93 | 10.523 | 12.542 | 11.049 | 1.00 | 21.99 |
| ATOM | 773 | CD1 | ILE 93 | 10.694 | 13.095 | 9.636 | 1.00 | 20.59 |
| ATOM | 774 | C | ILE 93 | 10.343 | 8.763 | 10.371 | 1.00 | 23.29 |
| ATOM | 775 | O | ILE 93 | 10.054 | 8.144 | 11.400 | 1.00 | 25.14 |
| ATOM | 776 | N | ASN 94 | 10.961 | 8.190 | 9.349 | 1.00 | 23.12 |
| ATOM | 777 | CA | ASN 94 | 11.299 | 6.782 | 9.413 | 1.00 | 22.49 |
| ATOM | 778 | CB | ASN 94 | 10.984 | 6.123 | 8.079 | 1.00 | 21.20 |
| ATOM | 779 | CG | ASN 94 | 9.531 | 6.256 | 7.711 | 1.00 | 22.72 |
| ATOM | 780 | OD1 | ASN 94 | 9.178 | 6.991 | 6.797 | 1.00 | 23.02 |
| ATOM | 781 | ND2 | ASN 94 | 8.671 | 5.551 | 8.440 | 1.00 | 25.40 |
| ATOM | 782 | C | ASN 94 | 12.729 | 6.483 | 9.816 | 1.00 | 21.72 |
| ATOM | 783 | O | ASN 94 | 13.650 | 7.245 | 9.519 | 1.00 | 21.83 |
| ATOM | 791 | N | ALA 96 | 15.288 | 2.723 | 10.867 | 1.00 | 20.30 |
| ATOM | 792 | CA | ALA 96 | 15.354 | 1.269 | 10.793 | 1.00 | 21.21 |
| ATOM | 793 | CB | ALA 96 | 16.782 | 0.817 | 10.661 | 1.00 | 20.69 |
| ATOM | 794 | C | ALA 96 | 14.720 | 0.631 | 12.019 | 1.00 | 22.49 |
| ATOM | 795 | O | ALA 96 | 13.951 | -0.322 | 11.897 | 1.00 | 26.57 |
| ATOM | 796 | N | GLY 97 | 15.043 | 1.151 | 13.195 | 1.00 | 20.75 |
| ATOM | 797 | CA | GLY 97 | 14.475 | 0.610 | 14.417 | 1.00 | 21.04 |
| ATOM | 798 | C | GLY 97 | 14.117 | 1.674 | 15.442 | 1.00 | 21.66 |
| ATOM | 799 | O | GLY 97 | 14.479 | 2.849 | 15.291 | 1.00 | 21.49 |
| ATOM | 800 | N | VAL 98 | 13.398 | 1.265 | 16.483 | 1.00 | 20.00 |
| ATOM | 801 | CA | VAL 98 | 12.996 | 2.180 | 17.542 | 1.00 | 19.82 |
| ATOM | 802 | CB | VAL 98 | 11.765 | 1.621 | 18.326 | 1.00 | 21.48 |
| ATOM | 803 | CG1 | VAL 98 | 11.474 | 2.482 | 19.553 | 1.00 | 17.19 |
| ATOM | 804 | CG2 | VAL 98 | 10.541 | 1.607 | 17.418 | 1.00 | 18.00 |
| ATOM | 805 | C | VAL 98 | 14.165 | 2.460 | 18.497 | 1.00 | 19.33 |
| ATOM | 806 | O | VAL 98 | 14.328 | 3.588 | 18.966 | 1.00 | 19.47 |
| ATOM | 807 | N | VAL 99 | 14.983 | 1.448 | 18.775 | 1.00 | 18.36 |
| ATOM | 808 | CA | VAL 99 | 16.139 | 1.638 | 19.652 | 1.00 | 20.24 |
| ATOM | 809 | CB | VAL 99 | 16.835 | 0.279 | 19.995 | 1.00 | 20.27 |
| ATOM | 810 | CG1 | VAL 99 | 18.193 | 0.526 | 20.630 | 1.00 | 18.24 |
| ATOM | 811 | CG2 | VAL 99 | 15.970 | -0.509 | 20.985 | 1.00 | 18.08 |
| ATOM | 812 | C | VAL 99 | 17.142 | 2.615 | 19.005 | 1.00 | 20.79 |
| ATOM | 813 | O | VAL 99 | 17.653 | 3.530 | 19.665 | 1.00 | 20.37 |
| ATOM | 814 | N | PRO100 | 17.442 | 2.433 | 17.709 | 1.00 | 19.99 |
| ATOM | 815 | CD | PRO100 | 17.096 | 1.321 | 16.803 | 1.00 | 20.82 |
| ATOM | 816 | CA | PRO100 | 18.384 | 3.366 | 17.075 | 1.00 | 19.69 |
| ATOM | 817 | CB | PRO100 | 18.477 | 2.844 | 15.641 | 1.00 | 19.12 |
| ATOM | 818 | CG | PRO100 | 18.236 | 1.353 | 15.803 | 1.00 | 20.07 |
| ATOM | 819 | C | PRO100 | 17.786 | 4.784 | 17.141 | 1.00 | 20.42 |
| ATOM | 820 | O | PRO100 | 18.497 | 5.770 | 17.349 | 1.00 | 17.25 |
| ATOM | 833 | N | PHE103 | 18.417 | 6.081 | 20.509 | 1.00 | 23.81 |
| ATOM | 834 | CA | PHE103 | 19.821 | 6.437 | 20.658 | 1.00 | 26.04 |
| ATOM | 835 | CB | PHE103 | 20.732 | 5.385 | 20.004 | 1.00 | 27.65 |
| ATOM | 836 | CG | PHE103 | 21.124 | 4.266 | 20.921 | 1.00 | 31.39 |
| ATOM | 837 | CD1 | PHE103 | 20.293 | 3.171 | 21.107 | 1.00 | 32.77 |
| ATOM | 838 | CD2 | PHE103 | 22.327 | 4.320 | 21.619 | 1.00 | 35.16 |
| ATOM | 839 | CE1 | PHE103 | 20.648 | 2.143 | 21.974 | 1.00 | 34.10 |
| ATOM | 840 | CE2 | PHE103 | 22.695 | 3.296 | 22.491 | 1.00 | 37.20 |
| ATOM | 841 | CZ | PHE103 | 21.848 | 2.203 | 22.669 | 1.00 | 35.91 |
| ATOM | 842 | C | PHE103 | 20.034 | 7.775 | 19.971 | 1.00 | 26.39 |
| ATOM | 843 | O | PHE103 | 20.861 | 8.591 | 20.391 | 1.00 | 26.76 |
| ATOM | 979 | N | VAL122 | 13.115 | -1.411 | 33.605 | 1.00 | 26.41 |
| ATOM | 980 | CA | VAL122 | 12.609 | -1.280 | 32.239 | 1.00 | 25.09 |
| ATOM | 981 | CB | VAL122 | 12.365 | 0.223 | 31.919 | 1.00 | 25.28 |
| ATOM | 982 | CG1 | VAL122 | 13.688 | 0.962 | 31.936 | 1.00 | 25.26 |
| ATOM | 983 | CG2 | VAL122 | 11.659 | 0.395 | 30.578 | 1.00 | 23.45 |
| ATOM | 984 | C | VAL122 | 13.562 | -1.863 | 31.191 | 1.00 | 23.77 |
| ATOM | 985 | O | VAL122 | 14.681 | -2.248 | 31.508 | 1.00 | 23.77 |
| ATOM | 986 | N | TYR123 | 13.089 | -1.915 | 29.945 | 1.00 | 24.39 |
| ATOM | 987 | CA | TYR123 | 13.832 | -2.411 | 28.780 | 1.00 | 24.09 |
| ATOM | 988 | CB | TYR123 | 13.111 | -1.927 | 27.518 | 1.00 | 23.60 |
| ATOM | 989 | CG | TYR123 | 13.708 | -2.374 | 26.209 | 1.00 | 23.67 |
| ATOM | 990 | CD1 | TYR123 | 14.018 | -3.712 | 25.982 | 1.00 | 25.33 |
| ATOM | 991 | CE1 | TYR123 | 14.523 | -4.134 | 24.749 | 1.00 | 24.54 |
| ATOM | 992 | CD2 | TYR123 | 13.920 | -1.462 | 25.174 | 1.00 | 23.19 |
| ATOM | 993 | CE2 | TYR123 | 14.417 | -1.874 | 23.938 | 1.00 | 23.70 |
| ATOM | 994 | CZ | TYR123 | 14.714 | -3.210 | 23.731 | 1.00 | 22.72 |
| ATOM | 995 | OH | TYR123 | 15.177 | -3.625 | 22.504 | 1.00 | 22.13 |
| ATOM | 996 | C | TYR123 | 15.282 | -1.917 | 28.811 | 1.00 | 24.31 |
| ATOM | 997 | O | TYR123 | 15.542 | -0.716 | 28.747 | 1.00 | 23.63 |
| ATOM | 998 | N | TYR124 | 16.221 | -2.858 | 28.880 | 1.00 | 25.99 |
| ATOM | 999 | CA | TYR124 | 17.649 | -2.551 | 29.004 | 1.00 | 28.34 |
| ATOM | 1000 | CB | TYR124 | 18.458 | -3.855 | 29.081 | 1.00 | 30.95 |
| ATOM | 1001 | CG | TYR124 | 18.258 | -4.813 | 27.927 | 1.00 | 34.27 |
| ATOM | 1002 | CD1 | TYR124 | 19.186 | -4.882 | 26.886 | 1.00 | 35.38 |
| ATOM | 1003 | CE1 | TYR124 | 19.019 | -5.777 | 25.831 | 1.00 | 35.75 |
| ATOM | 1004 | CD2 | TYR124 | 17.152 | -5.667 | 27.883 | 1.00 | 33.76 |
| ATOM | 1005 | CE2 | TYR124 | 16.976 | -6.563 | 26.834 | 1.00 | 34.93 |
| ATOM | 1006 | CZ | TYR124 | 17.913 | -6.613 | 25.811 | 1.00 | 36.06 |
| ATOM | 1007 | OH | TYR124 | 17.748 | -7.498 | 24.766 | 1.00 | 38.58 |
| ATOM | 1008 | C | TYR124 | 18.334 | -1.583 | 28.047 | 1.00 | 27.86 |
| ATOM | 1009 | O | TYR124 | 19.298 | -0.909 | 28.429 | 1.00 | 27.92 |
| ATOM | 1010 | N | PRO125 | 17.880 | -1.510 | 26.791 | 1.00 | 28.42 |
| ATOM | 1011 | CD | PRO125 | 17.059 | -2.473 | 26.039 | 1.00 | 30.26 |
| ATOM | 1012 | CA | PRO125 | 18.538 | -0.575 | 25.875 | 1.00 | 28.73 |
| ATOM | 1013 | CB | PRO125 | 17.827 | -0.842 | 24.554 | 1.00 | 29.49 |
| ATOM | 1014 | CG | PRO125 | 17.600 | -2.315 | 24.623 | 1.00 | 28.67 |
| ATOM | 1015 | C | PRO125 | 18.427 | 0.887 | 26.336 | 1.00 | 28.84 |
| ATOM | 1016 | O | PRO125 | 19.274 | 1.717 | 25.981 | 1.00 | 28.12 |
| ATOM | 1039 | N | MET128 | 21.389 | 1.773 | 28.066 | 1.00 | 31.71 |
| ATOM | 1040 | CA | MET128 | 22.463 | 1.837 | 27.089 | 1.00 | 32.39 |
| ATOM | 1041 | CB | MET128 | 22.380 | 0.624 | 26.160 | 1.00 | 36.35 |
| ATOM | 1042 | CG | MET128 | 22.728 | -0.688 | 26.857 | 1.00 | 39.05 |
| ATOM | 1043 | SD | MET128 | 22.445 | -2.157 | 25.854 | 1.00 | 44.93 |
| ATOM | 1044 | CE | MET128 | 23.662 | -1.917 | 24.536 | 1.00 | 43.28 |
| ATOM | 1045 | C | MET128 | 22.460 | 3.123 | 26.282 | 1.00 | 31.70 |
| ATOM | 1046 | O | MET128 | 23.497 | 3.767 | 26.129 | 1.00 | 30.91 |
| ATOM | 1069 | N | ASN132 | 26.010 | 4.816 | 27.402 | 1.00 | 29.89 |
| ATOM | 1070 | CA | ASN132 | 27.000 | 4.542 | 26.368 | 1.00 | 30.72 |
| ATOM | 1071 | CB | ASN132 | 26.488 | 3.475 | 25.398 | 1.00 | 29.64 |
| ATOM | 1072 | CG | ASN132 | 26.514 | 2.084 | 25.998 | 1.00 | 29.41 |
| ATOM | 1073 | OD1 | ASN132 | 26.816 | 1.904 | 27.182 | 1.00 | 28.33 |
| ATOM | 1074 | ND2 | ASN132 | 26.191 | 1.088 | 25.184 | 1.00 | 28.29 |
| ATOM | 1075 | C | ASN132 | 27.384 | 5.794 | 25.598 | 1.00 | 31.52 |
| ATOM | 1076 | O | ASN132 | 28.340 | 5.782 | 24.826 | 1.00 | 32.16 |
| ATOM | 1077 | N | GLN133 | 26.644 | 6.877 | 25.808 | 1.00 | 31.62 |
| ATOM | 1078 | CA | GLN133 | 26.930 | 8.120 | 25.112 | 1.00 | 30.35 |
| ATOM | 1079 | CB | GLN133 | 25.699 | 8.566 | 24.317 | 1.00 | 29.83 |
| ATOM | 1080 | CG | GLN133 | 25.139 | 7.479 | 23.390 | 1.00 | 29.49 |
| ATOM | 1081 | CD | GLN133 | 24.122 | 8.018 | 22.398 | 1.00 | 30.87 |
| ATOM | 1082 | OE1 | GLN133 | 24.475 | 8.736 | 21.463 | 1.00 | 33.39 |
| ATOM | 1083 | NE2 | GLN133 | 22.854 | 7.681 | 22.601 | 1.00 | 27.44 |
| ATOM | 1084 | C | GLN133 | 27.363 | 9.203 | 26.092 | 1.00 | 31.68 |
| ATOM | 1085 | O | GLN133 | 27.172 | 10.389 | 25.848 | 1.00 | 30.62 |
| ATOM | 1400 | N | CYS171 | 8.331 | 4.477 | 28.558 | 1.00 | 29.80 |
| ATOM | 1401 | CA | CYS171 | 7.988 | 3.226 | 27.896 | 1.00 | 27.89 |
| ATOM | 1402 | CB | CYS171 | 8.820 | 2.085 | 28.478 | 1.00 | 27.01 |
| ATOM | 1403 | SG | CYS171 | 8.838 | 0.595 | 27.458 | 1.00 | 25.24 |
| ATOM | 1404 | C | CYS171 | 6.497 | 3.004 | 28.174 | 1.00 | 27.54 |
| ATOM | 1405 | O | CYS171 | 6.094 | 2.824 | 29.327 | 1.00 | 26.83 |
| ATOM | 1406 | N | SER172 | 5.682 | 3.019 | 27.122 | 1.00 | 25.36 |
| ATOM | 1407 | CA | SER172 | 4.235 | 2.861 | 27.279 | 1.00 | 25.72 |
| ATOM | 1408 | CB | SER172 | 3.606 | 4.260 | 27.444 | 1.00 | 26.83 |
| ATOM | 1409 | OG | SER172 | 2.204 | 4.206 | 27.666 | 1.00 | 27.28 |
| ATOM | 1410 | C | SER172 | 3.571 | 2.126 | 26.100 | 1.00 | 24.15 |
| ATOM | 1411 | O | SER172 | 3.482 | 2.666 | 24.999 | 1.00 | 23.12 |
| ATOM | 1412 | N | PRO173 | 3.106 | 0.878 | 26.307 | 1.00 | 22.65 |
| ATOM | 1413 | CD | PRO173 | 2.294 | 0.238 | 25.259 | 1.00 | 20.23 |
| ATOM | 1414 | CA | PRO173 | 3.121 | 0.046 | 27.514 | 1.00 | 22.56 |
| ATOM | 1415 | CB | PRO173 | 2.498 | -1.250 | 27.031 | 1.00 | 22.80 |
| ATOM | 1416 | CG | PRO173 | 1.491 | -0.758 | 26.039 | 1.00 | 22.31 |
| ATOM | 1417 | C | PRO173 | 4.526 | -0.152 | 28.072 | 1.00 | 24.30 |
| ATOM | 1418 | O | PRO173 | 5.513 | -0.069 | 27.333 | 1.00 | 24.42 |
| ATOM | 1419 | N | HIS174 | 4.606 | -0.433 | 29.372 | 1.00 | 23.74 |
| ATOM | 1420 | CA | HIS174 | 5.885 | -0.591 | 30.049 | 1.00 | 25.08 |
| ATOM | 1421 | CB | HIS174 | 5.802 | 0.008 | 31.455 | 1.00 | 24.49 |
| ATOM | 1422 | CG | HIS174 | 7.138 | 0.216 | 32.097 | 1.00 | 23.35 |
| ATOM | 1423 | CD2 | HIS174 | 8.034 | -0.666 | 32.601 | 1.00 | 21.90 |
| ATOM | 1424 | ND1 | HIS174 | 7.711 | 1.463 | 32.233 | 1.00 | 23.11 |
| ATOM | 1425 | CE1 | HIS174 | 8.903 | 1.340 | 32.791 | 1.00 | 21.67 |
| ATOM | 1426 | NE2 | HIS174 | 9.124 | 0.057 | 33.024 | 1.00 | 20.80 |
| ATOM | 1427 | C | HIS174 | 6.480 | -1.999 | 30.149 | 1.00 | 27.64 |
| ATOM | 1428 | O | HIS174 | 6.072 | -2.821 | 30.973 | 1.00 | 27.16 |
| ATOM | 1429 | N | ASN175 | 7.469 | -2.253 | 29.307 | 1.00 | 29.75 |
| ATOM | 1430 | CA | ASN175 | 8.183 | -3.519 | 29.297 | 1.00 | 32.86 |
| ATOM | 1431 | CB | ASN175 | 8.849 | -3.681 | 27.918 | 1.00 | 34.28 |
| ATOM | 1432 | CG | ASN175 | 9.899 | -4.772 | 27.879 | 1.00 | 35.52 |
| ATOM | 1433 | OD1 | ASN175 | 9.771 | -5.814 | 28.525 | 1.00 | 35.99 |
| ATOM | 1434 | ND2 | ASN175 | 10.945 | -4.543 | 27.087 | 1.00 | 33.66 |
| ATOM | 1435 | C | ASN175 | 9.199 | -3.338 | 30.434 | 1.00 | 33.87 |
| ATOM | 1436 | O | ASN175 | 9.855 | -2.293 | 30.513 | 1.00 | 35.29 |
| ATOM | 1663 | N | ASP203 | 8.213 | 5.823 | 24.066 | 1.00 | 26.60 |
| ATOM | 1664 | CA | ASP203 | 8.387 | 4.638 | 23.240 | 1.00 | 26.96 |
| ATOM | 1665 | CB | ASP203 | 9.610 | 3.869 | 23.743 | 1.00 | 27.77 |
| ATOM | 1666 | CG | ASP203 | 9.819 | 2.549 | 23.039 | 1.00 | 24.93 |
| ATOM | 1667 | OD1 | ASP203 | 10.923 | 1.996 | 23.205 | 1.00 | 24.34 |
| ATOM | 1668 | OD2 | ASP203 | 8.904 | 2.062 | 22.341 | 1.00 | 25.76 |
| ATOM | 1669 | C | ASP203 | 7.119 | 3.822 | 23.413 | 1.00 | 28.14 |
| ATOM | 1670 | O | ASP203 | 6.919 | 3.194 | 24.448 | 1.00 | 30.03 |
| ATOM | 1671 | N | GLU204 | 6.260 | 3.842 | 22.397 | 1.00 | 28.83 |
| ATOM | 1672 | CA | GLU204 | 4.984 | 3.130 | 22.449 | 1.00 | 27.32 |
| ATOM | 1673 | CB | GLU204 | 3.841 | 4.132 | 22.234 | 1.00 | 24.98 |
| ATOM | 1674 | CG | GLU204 | 3.968 | 5.378 | 23.116 | 1.00 | 26.86 |
| ATOM | 1675 | CD | GLU204 | 2.734 | 6.277 | 23.098 | 1.00 | 28.98 |
| ATOM | 1676 | OE1 | GLU204 | 1.706 | 5.888 | 22.497 | 1.00 | 27.82 |
| ATOM | 1677 | OE2 | GLU204 | 2.794 | 7.373 | 23.700 | 1.00 | 28.65 |
| ATOM | 1678 | C | GLU204 | 4.903 | 2.001 | 21.420 | 1.00 | 27.71 |
| ATOM | 1679 | O | GLU204 | 3.827 | 1.697 | 20.904 | 1.00 | 27.46 |
| ATOM | 1680 | N | ILE205 | 6.042 | 1.372 | 21.141 | 1.00 | 28.16 |
| ATOM | 1681 | CA | ILE205 | 6.108 | 0.288 | 20.163 | 1.00 | 30.08 |
| ATOM | 1682 | CB | ILE205 | 7.572 | -0.222 | 20.019 | 1.00 | 31.86 |
| ATOM | 1683 | CG2 | ILE205 | 8.033 | -0.872 | 21.321 | 1.00 | 32.36 |
| ATOM | 1684 | CG1 | ILE205 | 7.674 | -1.195 | 18.842 | 1.00 | 30.81 |
| ATOM | 1685 | CD1 | ILE205 | 9.088 | -1.524 | 18.452 | 1.00 | 32.90 |
| ATOM | 1686 | C | ILE205 | 5.174 | -0.888 | 20.494 | 1.00 | 29.93 |
| ATOM | 1687 | O | ILE205 | 4.763 | -1.652 | 19.607 | 1.00 | 29.58 |
| ATOM | 1688 | N | HIS206 | 4.827 | -1.015 | 21.769 | 1.00 | 28.67 |
| ATOM | 1689 | CA | HIS206 | 3.946 | -2.082 | 22.223 | 1.00 | 28.85 |
| ATOM | 1690 | CB | HIS206 | 4.392 | -2.571 | 23.599 | 1.00 | 28.55 |
| ATOM | 1691 | CG | HIS206 | 5.539 | -3.520 | 23.558 | 1.00 | 26.73 |
| ATOM | 1692 | CD2 | HIS206 | 5.688 | -4.709 | 22.926 | 1.00 | 25.48 |
| ATOM | 1693 | ND1 | HIS206 | 6.714 | -3.299 | 24.241 | 1.00 | 26.13 |
| ATOM | 1694 | CE1 | HIS206 | 7.538 | -4.309 | 24.033 | 1.00 | 27.90 |
| ATOM | 1695 | NE2 | HIS206 | 6.936 | -5.178 | 23.236 | 1.00 | 26.82 |
| ATOM | 1696 | C | HIS206 | 2.488 | -1.677 | 22.324 | 1.00 | 28.14 |
| ATOM | 1697 | O | HIS206 | 1.656 | -2.486 | 22.741 | 1.00 | 26.27 |
| ATOM | 1939 | N | SER237 | 6.442 | -3.505 | 13.937 | 1.00 | 21.77 |
| ATOM | 1940 | CA | SER237 | 7.258 | -4.655 | 14.283 | 1.00 | 23.64 |
| ATOM | 1941 | CB | SER237 | 8.651 | -4.172 | 14.700 | 1.00 | 20.37 |
| ATOM | 1942 | OG | SER237 | 8.551 | -3.129 | 15.650 | 1.00 | 24.77 |
| ATOM | 1943 | C | SER237 | 6.695 | -5.627 | 15.327 | 1.00 | 24.81 |
| ATOM | 1944 | O | SER237 | 6.514 | -6.808 | 15.029 | 1.00 | 24.66 |
| ATOM | 1945 | N | LYS238 | 6.430 | -5.151 | 16.544 | 1.00 | 25.07 |
| ATOM | 1946 | CA | LYS238 | 5.907 | -6.030 | 17.580 | 1.00 | 24.40 |
| ATOM | 1947 | CB | LYS238 | 5.952 | -5.343 | 18.951 | 1.00 | 25.54 |
| ATOM | 1948 | CG | LYS238 | 7.346 | -5.224 | 19.562 | 1.00 | 28.52 |
| ATOM | 1949 | CD | LYS238 | 8.361 | -6.105 | 18.818 | 1.00 | 35.77 |
| ATOM | 1950 | CE | LYS238 | 9.724 | -6.066 | 19.457 | 1.00 | 36.68 |
| ATOM | 1951 | NZ | LYS238 | 10.182 | -4.665 | 19.541 | 1.00 | 41.95 |
| ATOM | 1952 | C | LYS238 | 4.487 | -6.488 | 17.285 | 1.00 | 25.71 |
| ATOM | 1953 | O | LYS238 | 4.149 | -7.656 | 17.485 | 1.00 | 26.92 |
| ATOM | 2017 | N | SER248 | 10.436 | 1.725 | 11.141 | 1.00 | 34.51 |
| ATOM | 2018 | CA | SER248 | 9.813 | 2.432 | 12.247 | 1.00 | 31.93 |
| ATOM | 2019 | CB | SER248 | 10.853 | 2.792 | 13.311 | 1.00 | 32.59 |
| ATOM | 2020 | OG | SER248 | 11.364 | 1.636 | 13.943 | 1.00 | 33.54 |
| ATOM | 2021 | C | SER248 | 9.143 | 3.705 | 11.760 | 1.00 | 30.54 |
| ATOM | 2022 | O | SER248 | 9.500 | 4.244 | 10.709 | 1.00 | 29.82 |
| ATOM | 2095 | N | ARG258 | 11.610 | 17.987 | 14.929 | 1.00 | 22.25 |
| ATOM | 2096 | CA | ARG258 | 11.642 | 16.974 | 13.875 | 1.00 | 23.68 |
| ATOM | 2097 | CB | ARG258 | 10.368 | 17.043 | 13.029 | 1.00 | 23.86 |
| ATOM | 2098 | CG | ARG258 | 10.281 | 15.953 | 11.972 | 1.00 | 26.34 |
| ATOM | 2099 | CD | ARG258 | 8.921 | 15.929 | 11.308 | 1.00 | 31.12 |
| ATOM | 2100 | NE | ARG258 | 8.571 | 17.215 | 10.709 | 1.00 | 35.18 |
| ATOM | 2101 | CZ | ARG258 | 7.520 | 17.949 | 11.064 | 1.00 | 36.02 |
| ATOM | 2102 | NH1 | ARG258 | 6.704 | 17.533 | 12.022 | 1.00 | 35.64 |
| ATOM | 2103 | NH2 | ARG258 | 7.279 | 19.098 | 10.452 | 1.00 | 38.72 |
| ATOM | 2104 | C | ARG258 | 12.877 | 17.109 | 12.964 | 1.00 | 23.38 |
| ATOM | 2105 | O | ARG258 | 13.570 | 16.123 | 12.679 | 1.00 | 22.12 |
| ATOM | 2106 | N | GLU259 | 13.153 | 18.326 | 12.512 | 1.00 | 20.36 |
| ATOM | 2107 | CA | GLU259 | 14.302 | 18.557 | 11.649 | 1.00 | 21.72 |
| ATOM | 2108 | CB | GLU259 | 14.331 | 20.017 | 11.206 | 1.00 | 20.16 |
| ATOM | 2109 | CG | GLU259 | 13.131 | 20.381 | 10.360 | 1.00 | 21.30 |
| ATOM | 2110 | CD | GLU259 | 13.001 | 21.861 | 10.130 | 1.00 | 22.23 |
| ATOM | 2111 | OE1 | GLU259 | 13.740 | 22.628 | 10.772 | 1.00 | 24.97 |
| ATOM | 2112 | OE2 | GLU259 | 12.151 | 22.261 | 9.310 | 1.00 | 25.38 |
| ATOM | 2113 | C | GLU259 | 15.618 | 18.179 | 12.327 | 1.00 | 23.32 |
| ATOM | 2114 | O | GLU259 | 16.516 | 17.643 | 11.680 | 1.00 | 22.79 |
| ATOM | 2137 | N | THR262 | 15.913 | 14.389 | 12.313 | 1.00 | 22.65 |
| ATOM | 2138 | CA | THR262 | 16.265 | 13.905 | 10.990 | 1.00 | 21.92 |
| ATOM | 2139 | CB | THR262 | 15.408 | 14.576 | 9.892 | 1.00 | 20.28 |
| ATOM | 2140 | OG1 | THR262 | 14.019 | 14.322 | 10.142 | 1.00 | 21.55 |
| ATOM | 2141 | CG2 | THR262 | 15.768 | 14.028 | 8.528 | 1.00 | 16.20 |
| ATOM | 2142 | C | THR262 | 17.735 | 14.148 | 10.681 | 1.00 | 24.40 |
| ATOM | 2143 | O | THR262 | 18.443 | 13.221 | 10.280 | 1.00 | 27.33 |
| ATOM | 2144 | N | LYS263 | 18.210 | 15.377 | 10.874 | 1.00 | 24.37 |
| ATOM | 2145 | CA | LYS263 | 19.608 | 15.663 | 10.578 | 1.00 | 24.61 |
| ATOM | 2146 | CB | LYS263 | 19.953 | 17.134 | 10.849 | 1.00 | 25.70 |
| ATOM | 2147 | CG | LYS263 | 20.320 | 17.457 | 12.283 | 1.00 | 28.50 |
| ATOM | 2148 | CD | LYS263 | 21.033 | 18.801 | 12.388 | 1.00 | 28.92 |
| ATOM | 2149 | CE | LYS263 | 21.235 | 19.183 | 13.840 | 1.00 | 27.76 |
| ATOM | 2150 | NZ | LYS263 | 21.802 | 20.543 | 13.979 | 1.00 | 32.81 |
| ATOM | 2151 | C | LYS263 | 20.513 | 14.759 | 11.406 | 1.00 | 23.54 |
| ATOM | 2152 | O | LYS263 | 21.557 | 14.313 | 10.932 | 1.00 | 20.99 |
| ATOM | 2159 | N | ARG265 | 19.680 | 11.734 | 12.583 | 1.00 | 23.00 |
| ATOM | 2160 | CA | ARG265 | 19.545 | 10.389 | 12.043 | 1.00 | 24.05 |
| ATOM | 2161 | CB | ARG265 | 18.098 | 10.083 | 11.685 | 1.00 | 25.34 |
| ATOM | 2162 | CG | ARG265 | 17.944 | 8.746 | 10.969 | 1.00 | 24.46 |
| ATOM | 2163 | CD | ARG265 | 16.727 | 8.726 | 10.081 | 1.00 | 21.84 |
| ATOM | 2164 | NE | ARG265 | 16.796 | 9.768 | 9.071 | 1.00 | 21.40 |
| ATOM | 2165 | CZ | ARG265 | 15.872 | 9.958 | 8.136 | 1.00 | 21.81 |
| ATOM | 2166 | NH1 | ARG265 | 14.806 | 9.171 | 8.086 | 1.00 | 22.44 |
| ATOM | 2167 | NH2 | ARG265 | 16.015 | 10.934 | 7.251 | 1.00 | 21.33 |
| ATOM | 2168 | C | ARG265 | 20.377 | 10.213 | 10.780 | 1.00 | 25.18 |
| ATOM | 2169 | O | ARG265 | 20.863 | 9.112 | 10.503 | 1.00 | 23.66 |
| ATOM | 2170 | N | ASP266 | 20.526 | 11.287 | 10.008 | 1.00 | 25.22 |
| ATOM | 2171 | CA | ASP266 | 21.292 | 11.218 | 8.768 | 1.00 | 28.03 |
| ATOM | 2172 | CB | ASP266 | 20.880 | 12.335 | 7.802 | 1.00 | 27.17 |
| ATOM | 2173 | CG | ASP266 | 19.486 | 12.137 | 7.252 | 1.00 | 29.27 |
| ATOM | 2174 | OD1 | ASP266 | 18.993 | 10.990 | 7.275 | 1.00 | 29.07 |
| ATOM | 2175 | OD2 | ASP266 | 18.889 | 13.128 | 6.782 | 1.00 | 31.48 |
| ATOM | 2176 | C | ASP266 | 22.798 | 11.263 | 8.978 | 1.00 | 28.97 |
| ATOM | 2177 | O | ASP266 | 23.564 | 10.983 | 8.056 | 1.00 | 30.53 |
| ATOM | 2200 | N | MET271 | 18.679 | 7.560 | 6.158 | 1.00 | 31.32 |
| ATOM | 2201 | CA | MET271 | 17.320 | 7.181 | 5.794 | 1.00 | 31.67 |
| ATOM | 2202 | CB | MET271 | 16.836 | 8.035 | 4.619 | 1.00 | 34.51 |
| ATOM | 2203 | CG | MET271 | 15.369 | 7.837 | 4.258 | 1.00 | 37.11 |
| ATOM | 2204 | SD | MET271 | 14.955 | 8.479 | 2.614 | 1.00 | 43.29 |
| ATOM | 2205 | CE | MET271 | 14.219 | 7.030 | 1.829 | 1.00 | 40.42 |
| ATOM | 2206 | C | MET271 | 17.242 | 5.709 | 5.404 | 1.00 | 30.73 |
| ATOM | 2207 | O | MET271 | 18.143 | 5.185 | 4.748 | 1.00 | 32.03 |
| ATOM | 2215 | N | PHE273 | 16.173 | 2.657 | 3.231 | 1.00 | 26.17 |
| ATOM | 2216 | CA | PHE273 | 15.802 | 2.495 | 1.826 | 1.00 | 26.40 |
| ATOM | 2217 | CB | PHE273 | 17.036 | 2.598 | 0.933 | 1.00 | 25.19 |
| ATOM | 2218 | CG | PHE273 | 17.743 | 3.916 | 1.021 | 1.00 | 26.86 |
| ATOM | 2219 | CD1 | PHE273 | 19.092 | 3.971 | 1.344 | 1.00 | 26.10 |
| ATOM | 2220 | CD2 | PHE273 | 17.067 | 5.107 | 0.765 | 1.00 | 27.63 |
| ATOM | 2221 | CE1 | PHE273 | 19.762 | 5.189 | 1.414 | 1.00 | 27.93 |
| ATOM | 2222 | CE2 | PHE273 | 17.730 | 6.335 | 0.832 | 1.00 | 27.99 |
| ATOM | 2223 | CZ | PHE273 | 19.079 | 6.376 | 1.158 | 1.00 | 27.54 |
| ATOM | 2224 | C | PHE273 | 15.141 | 1.140 | 1.588 | 1.00 | 26.63 |
| ATOM | 2225 | O | PHE273 | 14.745 | 0.816 | 0.469 | 1.00 | 26.84 |
| ATOM | 2252 | N | TYR278 | 8.662 | -0.840 | 3.899 | 1.00 | 23.64 |
| ATOM | 2253 | CA | TYR278 | 7.526 | 0.062 | 3.724 | 1.00 | 24.34 |
| ATOM | 2254 | CB | TYR278 | 7.813 | 1.108 | 2.641 | 1.00 | 22.56 |
| ATOM | 2255 | CG | TYR278 | 8.927 | 2.072 | 2.966 | 1.00 | 22.52 |
| ATOM | 2256 | CD1 | TYR278 | 10.195 | 1.906 | 2.423 | 1.00 | 25.51 |
| ATOM | 2257 | CE1 | TYR278 | 11.225 | 2.808 | 2.698 | 1.00 | 27.13 |
| ATOM | 2258 | CD2 | TYR278 | 8.711 | 3.162 | 3.799 | 1.00 | 24.74 |
| ATOM | 2259 | CE2 | TYR278 | 9.733 | 4.070 | 4.082 | 1.00 | 26.76 |
| ATOM | 2260 | CZ | TYR278 | 10.986 | 3.883 | 3.525 | 1.00 | 27.05 |
| ATOM | 2261 | OH | TYR278 | 12.004 | 4.771 | 3.787 | 1.00 | 30.94 |
| ATOM | 2262 | C | TYR278 | 6.258 | -0.699 | 3.351 | 1.00 | 24.63 |
| ATOM | 2263 | O | TYR278 | 5.181 | -0.422 | 3.882 | 1.00 | 25.58 |
| ATOM | 2793 | N | GLU343 | 19.247 | -18.017 | 37.193 | 1.00 | 36.87 |
| ATOM | 2794 | CA | GLU343 | 19.355 | -18.022 | 35.737 | 1.00 | 36.88 |
| ATOM | 2795 | CB | GLU343 | 20.253 | -16.870 | 35.292 | 1.00 | 36.37 |
| ATOM | 2796 | CG | GLU343 | 20.664 | -16.891 | 33.831 | 1.00 | 36.48 |
| ATOM | 2797 | CD | GLU343 | 21.548 | -15.715 | 33.487 | 1.00 | 34.71 |
| ATOM | 2798 | OE1 | GLU343 | 22.522 | -15.472 | 34.233 | 1.00 | 35.37 |
| ATOM | 2799 | OE2 | GLU343 | 21.276 | -15.035 | 32.479 | 1.00 | 32.72 |
| ATOM | 2800 | C | GLU343 | 19.941 | -19.358 | 35.286 | 1.00 | 36.89 |
| ATOM | 2801 | O | GLU343 | 19.480 | -19.963 | 34.313 | 1.00 | 35.47 |
| ATOM | 2830 | N | ILE347 | 18.033 | -22.009 | 32.792 | 1.00 | 35.20 |
| ATOM | 2831 | CA | ILE347 | 18.723 | -22.399 | 31.571 | 1.00 | 37.12 |
| ATOM | 2832 | CB | ILE347 | 20.035 | -21.591 | 31.394 | 1.00 | 38.99 |
| ATOM | 2833 | CG2 | ILE347 | 20.856 | -22.139 | 30.228 | 1.00 | 39.07 |
| ATOM | 2834 | CG1 | ILE347 | 19.697 | -20.120 | 31.156 | 1.00 | 39.17 |
| ATOM | 2835 | CD1 | ILE347 | 20.909 | -19.233 | 31.029 | 1.00 | 41.55 |
| ATOM | 2836 | C | ILE347 | 19.050 | -23.884 | 31.527 | 1.00 | 36.61 |
| ATOM | 2837 | O | ILE347 | 18.637 | -24.582 | 30.603 | 1.00 | 37.97 |
| ATOM | 2879 | N | PHE353 | 13.919 | -20.751 | 28.795 | 1.00 | 33.31 |
| ATOM | 2880 | CA | PHE353 | 14.646 | -19.510 | 28.564 | 1.00 | 31.56 |
| ATOM | 2881 | CB | PHE353 | 15.381 | -19.594 | 27.230 | 1.00 | 29.69 |
| ATOM | 2882 | CG | PHE353 | 16.368 | -20.714 | 27.161 | 1.00 | 26.35 |
| ATOM | 2883 | CD1 | PHE353 | 15.938 | -22.037 | 27.114 | 1.00 | 26.52 |
| ATOM | 2884 | CD2 | PHE353 | 17.729 | -20.453 | 27.172 | 1.00 | 23.47 |
| ATOM | 2885 | CE1 | PHE353 | 16.857 | -23.084 | 27.082 | 1.00 | 25.41 |
| ATOM | 2886 | CE2 | PHE353 | 18.655 | -21.493 | 27.140 | 1.00 | 24.47 |
| ATOM | 2887 | CZ | PHE353 | 18.218 | -22.809 | 27.097 | 1.00 | 22.72 |
| ATOM | 2888 | C | PHE353 | 13.821 | -18.236 | 28.612 | 1.00 | 31.37 |
| ATOM | 2889 | O | PHE353 | 13.126 | -17.893 | 27.660 | 1.00 | 29.50 |
| ATOM | 2901 | N | ASP355 | 13.753 | -13.958 | 30.029 | 1.00 | 32.92 |
| ATOM | 2902 | CA | ASP355 | 14.549 | -12.762 | 29.899 | 1.00 | 33.54 |
| ATOM | 2903 | CB | ASP355 | 13.764 | -11.666 | 29.177 | 1.00 | 35.73 |
| ATOM | 2904 | CG | ASP355 | 14.403 | -11.268 | 27.851 | 1.00 | 37.28 |
| ATOM | 2905 | OD1 | ASP355 | 14.726 | -12.174 | 27.050 | 1.00 | 33.90 |
| ATOM | 2906 | OD2 | ASP355 | 14.582 | -10.053 | 27.607 | 1.00 | 38.00 |
| ATOM | 2907 | C | ASP355 | 14.732 | -12.453 | 31.385 | 1.00 | 33.41 |
| ATOM | 2908 | O | ASP355 | 13.880 | -11.821 | 32.013 | 1.00 | 33.30 |
| ATOM | 2922 | N | TYR358 | 16.711 | -9.361 | 36.148 | 1.00 | 27.78 |
| ATOM | 2923 | CA | TYR358 | 17.792 | -8.467 | 36.538 | 1.00 | 28.47 |
| ATOM | 2924 | CB | TYR358 | 18.874 | -9.262 | 37.268 | 1.00 | 27.48 |
| ATOM | 2925 | CG | TYR358 | 19.761 | -10.083 | 36.373 | 1.00 | 27.06 |
| ATOM | 2926 | CD1 | TYR358 | 20.932 | -9.541 | 35.834 | 1.00 | 24.94 |
| ATOM | 2927 | CE1 | TYR358 | 21.765 | -10.301 | 35.023 | 1.00 | 23.67 |
| ATOM | 2928 | CD2 | TYR358 | 19.445 | -11.408 | 36.070 | 1.00 | 26.96 |
| ATOM | 2929 | CE2 | TYR358 | 20.279 | -12.179 | 35.253 | 1.00 | 22.96 |
| ATOM | 2930 | CZ | TYR358 | 21.432 | -11.620 | 34.741 | 1.00 | 24.06 |
| ATOM | 2931 | OH | TYR358 | 22.274 | -12.390 | 33.976 | 1.00 | 26.04 |
| ATOM | 2932 | C | TYR358 | 18.417 | -7.651 | 35.401 | 1.00 | 30.28 |
| ATOM | 2933 | O | TYR358 | 19.116 | -6.671 | 35.655 | 1.00 | 31.72 |
| ATOM | 2934 | N | ILE359 | 18.177 | -8.033 | 34.151 | 1.00 | 30.50 |
| ATOM | 2935 | CA | ILE359 | 18.749 | -7.264 | 33.047 | 1.00 | 30.70 |
| ATOM | 2936 | CB | ILE359 | 18.733 | -8.050 | 31.706 | 1.00 | 29.84 |
| ATOM | 2937 | CG2 | ILE359 | 19.481 | -9.382 | 31.871 | 1.00 | 30.44 |
| ATOM | 2938 | CG1 | ILE359 | 17.292 | -8.283 | 31.243 | 1.00 | 25.68 |
| ATOM | 2939 | CD1 | ILE359 | 17.185 | -8.938 | 29.894 | 1.00 | 19.66 |
| ATOM | 2940 | C | ILE359 | 17.986 | -5.951 | 32.854 | 1.00 | 32.09 |
| ATOM | 2941 | O | ILE359 | 18.461 | -5.046 | 32.166 | 1.00 | 32.82 |
| ATOM | 2942 | N | PHE360 | 16.806 | -5.846 | 33.464 | 1.00 | 32.09 |
| ATOM | 2943 | CA | PHE360 | 16.008 | -4.632 | 33.344 | 1.00 | 31.88 |
| ATOM | 2944 | CB | PHE360 | 14.519 | -4.962 | 33.250 | 1.00 | 29.02 |
| ATOM | 2945 | CG | PHE360 | 14.184 | -5.936 | 32.160 | 1.00 | 29.73 |
| ATOM | 2946 | CD1 | PHE360 | 14.292 | -7.303 | 32.375 | 1.00 | 30.51 |
| ATOM | 2947 | CD2 | PHE360 | 13.774 | -5.488 | 30.909 | 1.00 | 30.49 |
| ATOM | 2948 | CE1 | PHE360 | 13.995 | -8.215 | 31.358 | 1.00 | 32.98 |
| ATOM | 2949 | CE2 | PHE360 | 13.476 | -6.391 | 29.884 | 1.00 | 29.86 |
| ATOM | 2950 | CZ | PHE360 | 13.587 | -7.755 | 30.109 | 1.00 | 30.70 |
| ATOM | 2951 | C | PHE360 | 16.240 | -3.690 | 34.518 | 1.00 | 32.98 |
| ATOM | 2952 | O | PHE360 | 15.580 | -2.661 | 34.627 | 1.00 | 33.63 |
| ATOM | 3005 | N | ARG369 | 10.935 | -12.775 | 32.814 | 1.00 | 30.64 |
| ATOM | 3006 | CA | ARG369 | 9.980 | -12.297 | 31.819 | 1.00 | 28.99 |
| ATOM | 3007 | CB | ARG369 | 10.517 | -11.064 | 31.091 | 1.00 | 30.05 |
| ATOM | 3008 | CG | ARG369 | 9.420 | -10.213 | 30.463 | 1.00 | 31.16 |
| ATOM | 3009 | CD | ARG369 | 9.982 | -9.221 | 29.485 | 1.00 | 28.95 |
| ATOM | 3010 | NE | ARG369 | 10.523 | -9.901 | 28.320 | 1.00 | 32.19 |
| ATOM | 3011 | CZ | ARG369 | 11.170 | -9.290 | 27.336 | 1.00 | 33.28 |
| ATOM | 3012 | NH1 | ARG369 | 11.353 | -7.978 | 27.386 | 1.00 | 30.43 |
| ATOM | 3013 | NH2 | ARG369 | 11.631 | -9.993 | 26.306 | 1.00 | 31.01 |
| ATOM | 3014 | C | ARG369 | 9.718 | -13.414 | 30.810 | 1.00 | 27.27 |
| ATOM | 3015 | O | ARG369 | 10.628 | -13.871 | 30.125 | 1.00 | 26.09 |
| ATOM | 3050 | N | PRO375 | 2.125 | -20.796 | 20.731 | 1.00 | 27.96 |
| ATOM | 3051 | CD | PRO375 | 3.006 | -21.376 | 19.703 | 1.00 | 28.14 |
| ATOM | 3052 | CA | PRO375 | 0.914 | -21.606 | 20.908 | 1.00 | 28.64 |
| ATOM | 3053 | CB | PRO375 | 1.172 | -22.813 | 20.017 | 1.00 | 29.64 |
| ATOM | 3054 | CG | PRO375 | 2.047 | -22.245 | 18.921 | 1.00 | 29.57 |
| ATOM | 3055 | C | PRO375 | 0.774 | -21.992 | 22.378 | 1.00 | 30.33 |
| ATOM | 3056 | O | PRO375 | 1.770 | -22.122 | 23.097 | 1.00 | 30.32 |
| ATOM | 26939 | N1 | PLP500 | 11.375 | -0.633 | 22.701 | 1.00 | 35.52 |
| ATOM | 26940 | C2 | PLP500 | 10.806 | -1.477 | 23.645 | 1.00 | 36.15 |
| ATOM | 26941 | C2A | PLP500 | 10.169 | -0.798 | 24.812 | 1.00 | 35.08 |
| ATOM | 26942 | C3 | PLP500 | 10.858 | -2.920 | 23.376 | 1.00 | 37.22 |
| ATOM | 26943 | 03 | PLP500 | 10.280 | -3.671 | 24.381 | 1.00 | 38.68 |
| ATOM | 26944 | C4 | PLP500 | 11.460 | -3.408 | 22.205 | 1.00 | 38.19 |
| ATOM | 26945 | C4A | PLP500 | 11.651 | -4.922 | 22.072 | 1.00 | 41.10 |
| ATOM | 26946 | C5 | PLP500 | 12.053 | -2.388 | 21.251 | 1.00 | 37.45 |
| ATOM | 26947 | C6 | PLP500 | 11.973 | -1.035 | 21.561 | 1.00 | 35.32 |
| ATOM | 26948 | C5A | PLP500 | 12.830 | -2.827 | 19.948 | 1.00 | 35.86 |
| ATOM | 26949 | OP4 | PLP500 | 12.465 | -2.019 | 18.812 | 1.00 | 35.94 |
| ATOM | 26950 | P | PLP500 | 13.304 | -2.134 | 17.435 | 1.00 | 35.82 |
| ATOM | 26951 | OP1 | PLP500 | 14.659 | -1.622 | 17.748 | 1.00 | 33.45 |
| ATOM | 26952 | OP2 | PLP500 | 12.554 | -1.279 | 16.399 | 1.00 | 34.77 |
| ATOM | 26953 | OP3 | PLP500 | 13.250 | -3.561 | 17.038 | 1.00 | 37.69 |
| ATOM | 26954 | N4A | AVG500 | 12.852 | -5.365 | 22.743 | 1.00 | 42.11 |
| ATOM | 26955 | NI | AVG500 | 18.051 | -9.326 | 19.206 | 1.00 | 50.78 |
| ATOM | 26956 | CAI | AVG500 | 13.124 | -6.812 | 22.810 | 1.00 | 40.37 |
| ATOM | 26957 | CBI | AVG500 | 13.562 | -7.521 | 21.639 | 1.00 | 44.44 |
| ATOM | 26958 | CGI | AVG500 | 14.907 | -7.961 | 21.513 | 1.00 | 47.89 |
| ATOM | 26959 | CEI | AVG500 | 17.214 | -7.869 | 21.024 | 1.00 | 51.27 |
| ATOM | 26960 | CFI | AVG500 | 16.989 | -8.462 | 19.615 | 1.00 | 50.86 |
| ATOM | 26961 | CBC | AVG500 | 12.855 | -7.531 | 24.099 | 1.00 | 37.29 |
| ATOM | 26962 | OET | AVG500 | 16.027 | -7.036 | 21.422 | 1.00 | 50.57 |
| ATOM | 26963 | O2B | AVG500 | 13.131 | -8.894 | 24.250 | 1.00 | 35.68 |
| ATOM | 26964 | 03B | AVG500 | 12.341 | -6.872 | 25.101 | 1.00 | 35.50 |

Residues in the vicinity of the active site. Column 4 indicates the residue type, with AVG as an abbreviation for aminoethoxyvinylglycine. The PLP cofactor is in a Schiff-base linkage with the α-amino group of the AVG. Column 5 indicates the residue number, with residue number 500 assigned to the covalent adduct of PLP and AVG. For residue numbers greater than 99, column 5 appears glued to column 4. Column 3 indicates the atom type, with N4A indicating the α-amino group that forms part of the Schiff-base linkage to the cofactor, and with NI indicating the terminal amino group of AVG. 02B and 03B in AVG are the carboxylate oxygens linked to the carboxylate carbon CBC. Columns 6, 7, 8 give the x, y, z coordinates in Ångstrom units. Column 9 indicates the occupancy, and column 10 indicates the B-factor.

### References

Alexeev, D., Alexeeva, M., Baxter, R. L., Campopiano, D., Webster,S. P. and Sawyer, L. (1998) The crystal structure of 8-amino-7-oxononoate synthase: a bacterial PLP-dependent Acyl-CoA-condensing enzyme. *J. Mol. Biol*., **284,** 401-419.
Beauchamp, R. O., Bus, J. S., Popp, J. A., Boreiko, C. J. and Andjelkovich, D. A. (1984) A critical review of the literature on hydrogen sulfide toxicity. *Crit. Rev. Toxicol*., **13,** 25-97.
Blankenfeldt, W., Nowicki, C., Hunter, G. R., Montemartini-Kalisz, M., Kalisz, H. M. and Hecht, H. J. (1999) Crystal structure of *Trypanosoma cruzi* tyrosine aminotransferase: substrate specificity is influenced by cofactor binding mode. *Protein Sci.,* **8,** 2406-2417.
Brünger, A. T., Adams, P. D., Clore, G. M., Delano, W. L., Gros, P., Grosse-Kunstleve, R. W., Jiang, J. S., Kuszewski, J., Nilges, N., Pannu, N. S., Read, R. J., Rice, L. M., Simonson, T. and Warren, G. L. (1998) Crystallography and NMR system: A new software suite for macromolecular structure determination. *Acta Crystallogr.,* **D54,** 905-921.
CCP4, Collaborative Computational Project 4 (1994) The CCP4 suite: programs for protein crystallography. *Acta Crystallogr.,* **D50,** 760-763.
Chu, L. and Holt, S. C. (1994) Purification and characterization of a 45 kDa hemolysin from *Treponema denticola* ATCC 35404. *Microb. Pathog.,* **16,** 197-212.
Chu, L., Kennell, W. and Holt, S. C. (1994) Characterization of hemolysis and hemoxidation activities of *Treponema denticola. Microb. Pathog.,* **16,** 183-195.
Chu, L., Burgum, A., Kolodrubetz, D. and Holt, S. C. (1995) The 46-kilodalton-hemolysin gene from *Treponema denticola* encodes a novel hemolysin homologous to aminotransferases. *Infect. Immun*., **63,** 4448-4455.
Chu, L., Ebersole, J. L., Kurzban, G. P. and Holt, S. C. (1997) Cystalysin, a 46-kilodalton cysteine desulfhydrase *Treponema denticola,* with hemolytic and hemoxidative activities. *Infect. Immun*., **65,** 3231-3238.
Clausen, T., Huber, R., Laber, B., Pohlenz, H. D. and Messerschmidt, A. (1996) Crystal structure of the pyridoxal 5'-phosphate dependent cystathionine -lyase from *Escherichia coli* at 1.83 Å. *J. Mol. Biol*., **262,** 202-224.
Clausen, T., Huber, R., Messerschmidt, A., Pohlenz, H.D. and Laber, B. (1997) Slow-binding inhibition of *Escherichia coli* cystathionine -lyase by *L*-aminoethoxyvinylglycine: a kinetic and X-ray study. *Biochemistry,* **36,** 12633-12643.
Clausen, T., Schlegel, A., Preist, R., Schneider, E., Steegborn, C., Chang, Y. S., Haase, A., Bourenkov, G. P., Bartunik, H. and Boos, W. (2000) X-ray structure of MalY from *Escherichia coli*: a pyridoxal 5'-phosphate-dependent enzyme acting as a modulator in *mal* gene expression. *EMBO J.* **19**, 831-842.
Delepelaire, P. and Wandersman, C. (1990) Protein secretion in gram-negative bacteria. The extracellular metalloprotease B from *Erwinia chrysanthemi* contains a C-terminal secretion signal analogous to that of *Escherichia coli* alpha hemolysin. *J. Biol. Chem.,* **265,** 17118-17125.
Dwivedi, C. M., Ragin, R. C. and Uren, J. R. (1982) Cloning, purification and characterization of-cystathionase from *E*. *coli. Biochemistry,* **21,** 3064-3069.
Engh, R. A. and Huber, R. (1991) Accurate bond and angle parameters for X-ray protein-structure refinement. *Acta Crystallogr*., **A47,** 392-400.
Ferrell, Jr. J. E., Lee, K. J. and Huestis, W. H. (1985) Membrane bilayer balance and erythrocyte shape: a quantitative assessment. *Biochemistry,* **24,** 2849-2857.
Frischman, D. and Argos, P. (1995) Knowledge-based secondary structure assignment. *Proteins,* **23,** 566-579.
Gentry-Weeks, C. R., Keith, J. M. and Thompson, J. (1993) Toxicity of *Bordetella avium* -cystathionase toward MC3T3-E1 osteogenic cells. *J. Bio. Chem*., **268,** 7298-7314.
Hayashi, H. and Kagamiyama, H. (1997) Transient-state kinetics of the reaction of aspartate aminotransferase with aspartate at low pH reveals dual routes in the enzyme-substrate association process. *Biochemistry,* **36,** 13558-13569.
Holm, L. and Sander, C. (1993) Protein structure comparison by alignment of distance matrices. *J. Mol. Biol*., **233,** 123-138.
Jansonius, J. N. (1998) Structure, evolution and action of vitamin B-6-dependent enzymes. *Curr. Opin. Struct. Biol*., **8,** 759-769.
Jones, T. A., Zou, J. Y. Cowan, S. W. and Kjelgaard, M. (1991) Improved methods for building protein models in electron density maps and location of errors in these models. *Acta Crystallogr*., **A47,** 110-119.
Jones, T. A. (1992) a, yaap, asap, @**? A set of averaging programs. In *Molecular Replacement (CCP4),* SERC Daresbury Laboratory, Warrington, UK, pp. 92-105.
Jones, S. and Thornton, J. M. (1996) Principles of protein-protein interactions derived from structural studies. *Proc. Natl Acad. Sci. USA,* **93,**13-20.
Kabsch, W. and Sander, C. (1983) Dictionary of protein secondary structure: pattern recognition of hydrogen bonded and geometrical features. *Biopolymers*, **22,** 2577-2637.
Käck, H., Sandmark, J., Gibson, K., Schneider, G. and Lindqvist, Y. (1999) Crystal structure of diaminopelargonic acid synthase: evolutionary relationships between pyridoxal 5'-phosphate-dependent enzymes. *J. Mol. Biol*., **291,** 857-876.
Karunakaran, T. and Holt, S. C. (1994) Cloning and expression of hemolysin genes from *Treponema denticola* strains ATCC 35404 (TD-4) and human clinical isolate GM-1 in *Escherichia coli. Microb. Pathog.,* **16,** 337-348.
Kleywegt, G.T. (1996) Use of non-crystallographic symmetry in protein structure refinement. *Acta Crystallogr*., **D52,** 842-857.
Kurzban, G. P., Chu, L., Ebersole, J. L. and Holt, S. C. (1999) Sulfhemoglobin formation in human erythrocytes by cystalysin, an *L*-cysteine desulfhydrase form *Treponema denticola. Oral Microbiol. Immunol.,* **14,** 153-164.
Laskowski, R. A., MacArthur, M. W., Mass, S. D. and Thornton, J. M. (1993) PROCHECK: a program to check the stereochemical quality of protein structures. *J. Appl. Cryst.,* **26,** 283-291.
Leibrecht, I. and Kessler, D. (1997) A novel *L*-cysteine/cystine C-S-Lyase directing [2Fe-2S] cluster formation of *Synechocystis* ferredoxin. *J. Biol. Chem*., **272,** 10442-10447.
Luzzatti, V. (1952) Traitement statistique des erreurs das la détermination des structures cristallines. *Acta Crystallogr*., **A5,** 802-810.
Metha, K., Hale, T. I. and Christen, P. (1989) Evolutionary relationships among aminotransferases. *Eur. J. Biochem.,* **186,** 249-253.
Metha, P. K., Hale, T. I. and Christen, P. (1993) Aminotransferases: demonstration of homology an division into evolutionary subgroups. *Eur. J. Biochem.,* **214,** 249-253.
Morrison, J. F. and Walsh, C. T. (1988). The behavior and significance of slow-binding inhibitors. *Adv. Enzymol. Relat. Areas Mol. Biol*., **61**, 201-301. Moxness, M. S., Brunauer, L. S. and Huestis, W. H. (1996) Hemoglobin oxidation products extract phospholipids from the membrane of human erythrocytes. *Biochemistry, 35,* 7181-7187.
Nakai, T., Okada, K., Akutsu, S., Miyahara, I., Kawaguchi, S., Kato, R., Kuramitsu, S. and Hirotsu, K. (1999) Structure of *Thermus thermophilus* Hb8 aspartate aminotransferase and its complex with maleate. *Biochemistry,* **38,** 2431-2424.
Navaza, J. (1994) AMoRe: An automated package for molecular replacement. *Acta Crystallogr*., **A50**, 157-163.
Ng, W. and Tonzetich, J. (1984) Effect of hydrogen sulfide and methyl mercaptan on the permeability of oral mucosa. *J. Dent. Res.,* **63,** 994-997. Okamoto, A., Kato, R., Masui, R., Yamagishi, A., Oshima, T. and Kuramitsu, S. (1996) *J. Biochem.,* **119,** 135-144.
Oliver, D. (1985) Protein secretion in *Escherichia coli. Annu. Rev. Microbiol.,* **39,** 615-648.
Otwinowski, Z. and Minor, W. (1996) Processing of X-ray diffraction data collected in oscillation mode. *Methods Enzymol*., **276,** 307-326.
Owens, L. D., Guggenheim, S. and Hilton, J. L. (1968) Rhizobium synthesized phytotoxin: an inhibitor of -cystathionase in *Salmonella typhimurium. Biochim. Biophys. Acta*, **158,** 219-225.
Persson, S., Edlund, M. B., Claesson, R. and Carlsson, J. (1990) The formation of hydrogen sulfide and methyl mercaptan by oral bacteria. *Oral Microb. Immunol*., ***5**,* 195-201.
Pruess, D. L., Scannell, J. P., Kellet, M., Ax, H. A., Janecek, J., Williams, T. H., Stempel, A. and Berger, J. (1974) Antimetabolites produced by microorganisms. X. *L*-2-amino-4-(2-aminoethoxy)-trans-3-butenoic acid. *J. Antibiot*., **27,** 229-233.
Ramachandran, G. N. and Sasisekharan, V. (1968) Conformation of polypeptides and proteins. *Adv. Protein Chem*., **23,** 283-437.
Reuter, W. and Wehrmeyer, W. (1988) Core substructure in *mastigocladus laminosus* phycobilisomes I. Microheterogeneity in two of three allophycocyanin core complexes. *Arch. Microbiol*., **150,** 534-540.
Rhee, S., Silva, M. M., Hyde, C. C., Rogers, P. H., Metzler, C. M., Metzler, D. E. and Arnone, A. (1997) Refinement and comparisons of the crystal structures of pig cytosolic aspartate aminotransferase and its complex with 2-methylaspartate. *J. Biol. Chem.,* **272,** 17293-17302.
Song, L., Hobaugh, M. R., Shustak, C., Cheley, S., Bayley, H. and Gouaux, J. E. (1996) Structure of staphylococcal -hemolysin, a heptameric transmembrane pore. *Science,* **274,** 1859-1866.
Sung, M., Tanizawa, K., Tanaka, H., Kuramitsu, S., Kagamiyama, H., Hirotsu, K., Higuchi, T. and Soda, K. (1991) Thermostable aspartate aminotransferase from a thermophilic *Bacillus sp* gene. Cloning sequence determination and preliminary x-ray characterization. *J. Biol. Chem.,* **266,** 2567-2572.
Toohey, J. I. (1989) Sulfane sulfur in biological systems: a possible regulatory role. *Biochem. J.,* **264,** 625-632.
Valentine, W. N., Toohey, J. I., Paglia, D. E., Nakatani, M. and Brockway, R. A. (1987) Modification of erythrocyte enzyme activities by persulfides and methanethiol: possible regulatory role. *Proc. Natl Acad. Sci. USA*, **84,** 1394-1398.
Watson, R. J. and Rastogi, V. K. (1993) Cloning and nucleotide sequencing of *Rhizobium meliloti* aminotransferase genes: An aspartate aminotransferase required for symbiotic nitrogen fixation is atypical. *J. Bacteriol*., **175,** 1919-1928.
Yano, T., Kuramitsu, S., Tanase, S., Morino, Y. and Kagamiyama, H. (1992) Role of Asp222 in the catalytic mechanism of *Escherichia coli* aspartate aminotransferase. *J*. *Biol. Chem.,* **267,** 12506-12501.
Zdych, E., Peist, R., Reidl, J. and Boos, W. (1995) MalY of *Escherichia coli* is an enzyme with the activity of a beta-*C-S* lyase (cystathionase). *J. Bacteriol*., **177,** 5035-5039.

## Claims

1. Crystalline preparation of cystalysin of *Treponema denticola* as shown in Fig. 1.

2. Crystalline preparation of a cystalysin/L-aminoethoxyvinglglycine complex as shown in Fig. 4.

3. Use of crystalline preparations according to claims 1 or 2 to obtain crystal structure data for the design and/or indentification of inhibitors of cystalysin.

4. Use of crystal structure data obtained according to claim 3 for the design and/or identification and/or preparation of inhibitors of cystalysin.

5. Use according to claim 4 wherein a computer-aided modeling program is used for the design of inhibitor molecules.

6. Cystalysin inhibitor, **characterized in that** it is able to specifically bind to and/or interact with the active site of cystalysin and/or with the site where dimerization occurs.

7. Cystalysin inhibitor according to claim 6, **characterized in that** it specifically binds to and/or interacts with the active site of cystalysin and either cannot be cleaved by the enzyme or is cleaved to expose a reactive group that inactivates the enzyme.

8. Cystalysin inhibitor according to claim 6or 7, **characterized in that** it is able to specifically bind to and/or interact with one or more of the following amino acids: Leu17, Val38, Ala39, Asp40, Tyr64*, Val98, Val99, Tyr123, Tyr124, Phe162, Cys171, Pro173, Asn175, Asp203, His206, Ser237, Lys238, Phe273*, Ile356, Phe360 and/or Arg369.

9. Cystalysin inhibitor according to any one of claims 6 to 8, **characterized in that** it binds reversibly to cystalysin via non-covalent or hydrolytically labile covalent interaction.

10. Cystalysin inhibitor according to claim 9, **characterized in that** it binds via salt bridges or hydrogen bonds.

11. Cystalysin inhibitor according to any one of claims 6 to 10, **characterized in that** it contains a backbone chain to which one or more groups that mediate the binding or interaction are attached.

12. Cystalysin inhbitor according to claim 11, **characterized in that** the backbone chain is a polypeptide or a synthetic organic molecule.

13. Cystalysin inhbitor according to claim 12, **characterized in that** the polypeptide backbone chain contains 1-8 amino acids or the synthetic organic backbone chain has a length corresponding to such polypeptide chain length.

14. Cystalysin inhbitor according to any one of claims 11 to 13, **characterized in that** the synthetic organic backbone chain is an alkyl chain optionally containing heteroatoms, substitutions, double or triple bonds and/or ring systems optionally containing heteroatoms to reduce the flexibility of the chain, but only to an extent that allows the functional groups of an inhibitor molecule to adjust to the appropriate anchor groups of cystalysin.

15. Cystalysin inhibitor according to any one of the claims 6 to 14, **characterized in that** it contains a carboxylate group or the like that anchors the molecule to Arg369.

16. Cystalysin inhibitor according to 15, **characterized in that** it has an amino group in α-position with respect to the carboxylate group.

17. Cystalysin inhibitor according to 15 or 16, **characterized in that** it is an amino acid analogue.

18. Cystalysin inhibitor according any of the claims 15 to 17, **characterized in that** it contains a β,γ-double bond.

19. Cystalysin inhibitor according to 15, **characterized in that** does not contain an amino group in α-position with respect to the carboxylate group.

20. Cystalysin inhibitor according to any of the claims 15-19, **characterized in that** it contains bulky groups 5-9 Å and/or 9-13 Å away from the carboxylate group or the like that anchors the molecule to R369.

21. Cystalysin inhibitor according to claim 20, wherein the bulky groups anchor the molecule to the pockets indicated in Fig.6.

22. Pharmaceutical composition, **characterized in that** it contains a cystalysin inhibitor according to any one of claims 6-21.

23. Pharmaceutical composition according to claim 22, **characterized in that** it additionally contains pharmaceutically acceptable carriers or adjuvant substances.

24. Use of a pharmaceutical composition according to claims 22 or 23 for the treatment and/or prevention of periodontal diseases, especially periodontitis and/or cardiovascular diseases that occur after a primary periodontal disease.
